# EUROPEAN PATENT APPLICATION

(11) **EP 4 431 501 A1**
(43) Date of publication of application: **18.09.2024**
(21) Application number: 22892086.4
(22) Date of filing: 11.11.2022
(51) Int. Cl.: C07D 319/18, A61K 31/4025, A61P 43/00

(54) **PHARMACEUTICALLY ACCEPTABLE SALT OF ELIGLUSTAT AND CRYSTAL FORM THEREOF**

(30) Priority: 12.11.2021 CN 202111342542
(71) Applicant: Sperogenix (Shanghai) Medtech Co., Ltd., Shanghai 200032 (CN)
(72) Inventor: LIU, Hanlan, Lexington, Massachusetts 02420 (US); WU, Yansheng, Maynard, Massachusetts 01754 (US); GUO, Chunzhe, Beijing 100027 (CN); WANG, Xiaohui, Beijing 100027 (CN); YAN, Zhiyu, Shanghai 200032 (CN)
(74) Representative: Cabinet Becker et Associés
(86) International application number: PCT/CN2022/131323
(87) International publication number: WO 2023/083293

(57) **Abstract**

The present invention provides multiple pharmaceutically acceptable salts of eliglustat, including naphthalene disulfonate, oxalate, glutarate, mucate, and multiple crystalline forms thereof, and also provides pharmaceutical compositions containing the same, the preparation methods thereof, and the uses for treating Gaucher's disease, Fabry's disease, and polycystic kidney disease.

## Description

### FIELD OF THE INVENTION

The present invention relates to pharmaceutically acceptable salts of Eliglustat and the crystalline forms thereof, a preparation method, a pharmaceutical composition comprising the crystalline forms and the use thereof, and belongs to the field of pharmaceutical technology.

### DESCRIPTION OF THE RELATED ART

Eliglustat, with the chemical name of N-[(1R,2R)-1-(2,3-dihydro-1,4-benzodioxin-6-yl)-2-hydroxy-1-(1-pyrrolidinemethyl)ethyl ]octanamide, is a potent and highly specific ceramide analog inhibitor that reduces the production of glucosylceramide by targeting glucosylceramide synthase. Cerdelga^{®}, an eliglustat capsule, was approved by the US FDA in 2014 for the long-term treatment of first-line treatment of adult patients with Type I Gaucher's disease. Clinical studies have shown that the combination of Eliglustat Capsules Cerdelga^{®} with CYP2D6 and CYP3A4 inhibitor drugs may significantly increase drug exposure and cause PR, QTc, and/or the prolongation of QRS cardiac intervals, leading to arrhythmia; pharmacokinetic/pharmacodynamic models predicted mean values increase in PR, QRS, and QTcF intervals of 22 (26), 7 (10), and 13 (19) milliseconds when the plasma concentrations of eliglustat reaches 500 ng/mL. With regard to patients with poor metabolism of CYP2D6, compared with the dosage of 84 mg taken orally twice a day for CYP2D6 extensive and intermediate metabolizers, the dosage of eliglustat capsule is reduced to 84 mg orally once a day to avoid the side effects of arrhythmia in patients with poor metabolism of CYP2D6. Based on the above factors, there are many inconveniences in clinical use of Cerdelga^{®}, an eliglustat capsule on the market.

Eliglustat and the preparation method thereof are described in US6916802B2, which briefly described that "pharmaceutically acceptable salts" are, e.g., inorganic acids, such as sulfuric acid, hydrochloric acid, phosphoric acid, etc., or organic acids, such as acetates. There is no further studies conducted on the physicochemical properties of pharmaceutically acceptable salts, nor on the crystalline forms of the salts.

The forms of physiologically acceptable salts of eliglustat are simply described in US7196205B2. There is no further studies conducted on the physicochemical properties of pharmaceutically acceptable salts, nor on the crystalline forms of the salts.

Affected by the inherent properties of eliglustat free base, there are technical obstacles in obtaining stable solid forms of salts and the crystalline forms thereof. For example, WO2011066352A1 describes that, the salts of eliglustat include citrate, malate, fumarate, methanesulfonate and acetate, but these salts cannot be obtained in solid form; although the hydrochloride salt and the 1:1 tartrate salt are available in solid form, both are not crystalline and are too hygroscopic for formulation. Eliglustat hemitartrate is easier to formulate and synthesize than the free base and other salts. Said patent specifically discloses that eliglustat hemitartrate and the crystalline form thereof are crystalline, non-hygroscopic, water-soluble, have better fluidity than corresponding free bases and other salts, are suitable for large-scale preparation, and have main X-ray powder diffraction peaks at 2θ angles of 5.1°, 6.6°, 10.7°, 1 1.0°, 15.9°, and 21.7°. Another L-hemitartrate crystalline form of eliglustat is described in CN107445938A, wherein the X-ray powder diffraction has main characteristic peaks at 2θ angles of 10.2°, 12.4°, 13.6°, 14.9°, 20.1°, and 22.1°, and the DSC spectrum shows an endothermic peak at 161°C~162°C.

The present invention provides a new eliglustat salt and the crystalline form thereof, which can exist in a stable solid form. Eliglustat 1,5-naphthalene disulfonate and the crystalline form thereof have lower hygroscopicity over eliglustat hemitartrate crystalline form A, and the solubility difference in water and simulated gastric fluid is small. It is expected to have a flatter plasma drug concentration after oral administration, thereby reducing the adverse responses of arrhythmia caused by an increase in exposure dose.

### SUMMARY

The present invention provides a pharmaceutically acceptable salt, solvate and/or crystalline form of eliglustat that can exist in a stable solid form.

In some embodiments, the solubility of pharmaceutically acceptable salts, solvates and or crystalline forms of eliglustat in water and simulated gastric fluid is less than or equal to 6.0 mg/mL.

In some embodiments, pharmaceutically acceptable salts of eliglustat are naphthalene disulfonate, mucate, glutarate.

In some embodiments, the naphthalene disulfonate of eliglustat can be selected from the group consisting of 1,5-naphthalene disulfonate, 1,6-naphthalene disulfonate, 1,7-naphthalene disulfonate, 2,6-naphthalene disulfonate, 2,7-naphthalene disulfonate.

In some embodiments, the molar ratio of 1,5-naphthalene disulfonic acid and eliglustat is 1:1 or 1:2.

In some embodiments, the pharmaceutically acceptable salts of eliglustat are the hydrate or unsolvate of 1,5-naphthalene disulfonate. Hydrates of 1,5-naphthalene disulfonate include hemihydrate, monohydrate, and dihydrate.

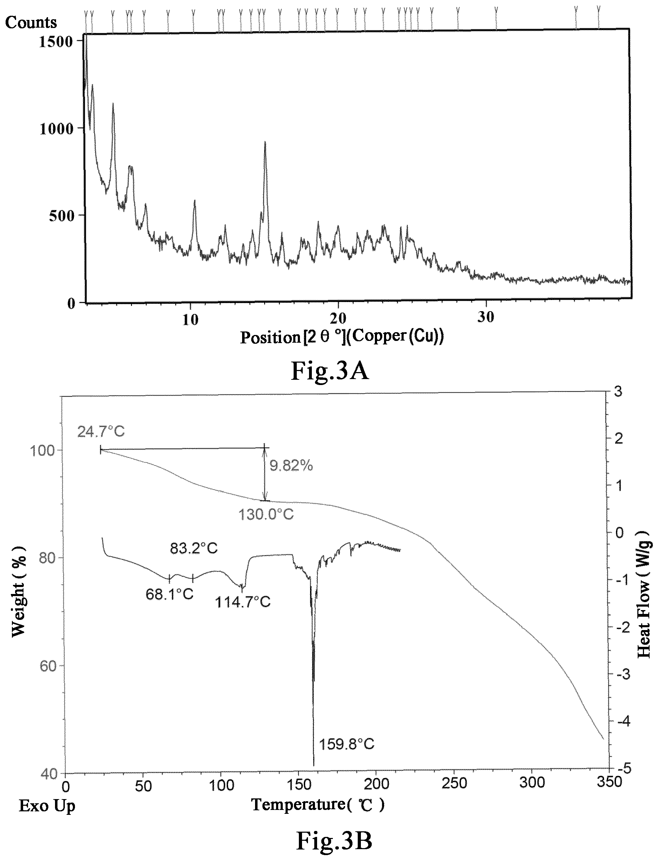

In some embodiments, there is provided a crystalline form D of eliglustat 1,5-naphthalene disulfonate with X-ray powder diffraction peaks at 2θ angles (± 0.2°) of 4.9°, 5.9°, and 18.7°. The crystalline form D of the eliglustat 1,5-naphthalene disulfonate further comprises X-ray powder diffraction peaks at 2θ angles (± 0.2°) of 7.0°, 10.4°, and 24.7°. The crystalline form D of the eliglustat 1,5-naphthalene disulfonate further comprises X-ray powder diffraction peaks at 2θ angles (± 0.2°) of 14.2° and 16.2°. The crystalline form D of the eliglustat 1,5-naphthalene disulfonate has an X-ray powder diffraction pattern that is substantially similar to Fig. 3A.

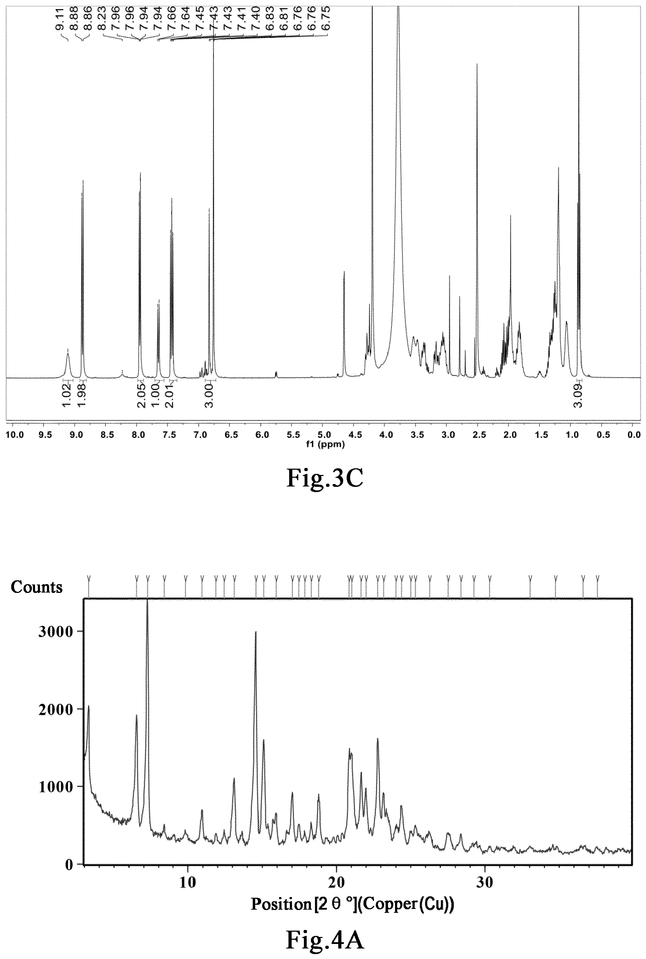

In some embodiments, there is provided a crystalline form B of eliglustat 1,5-naphthalene disulfonate with X-ray powder diffraction peaks at 2θ angles (± 0.2°) of 7.3°, 14.6°, and 6.5°. The crystalline form B of the eliglustat 1,5-naphthalene disulfonate further comprises X-ray powder diffraction peaks at 2θ angles (± 0.2°) of 22.8°, 21.0°, and 20.8°. The crystalline form B of the eliglustat 1,5-naphthalene disulfonate further comprises X-ray powder diffraction peaks at 2θ angles (± 0.2°) of 13.1°, 3.3° and 15.1°. The crystalline form B of the eliglustat 1,5-naphthalene disulfonate has an X-ray powder diffraction pattern that is substantially similar to Fig. 4A.

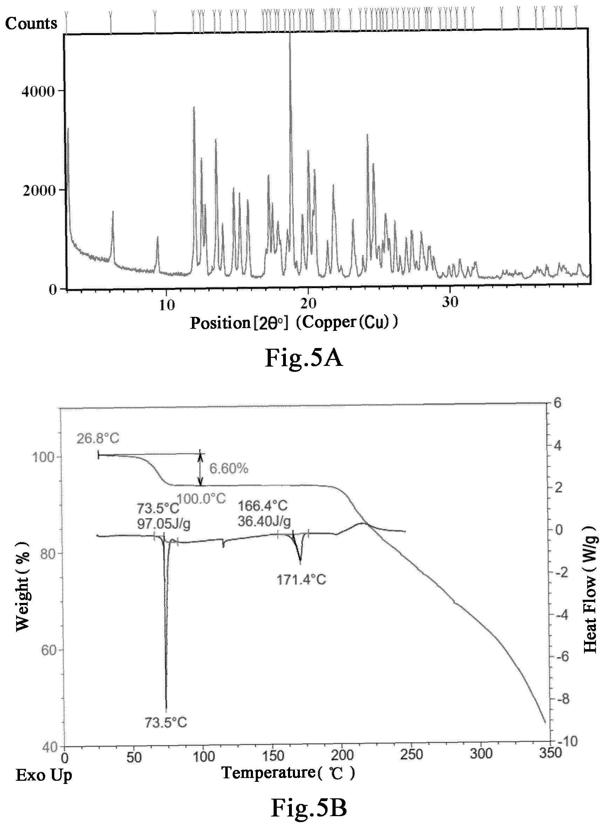

**In** some embodiments, there is provided a crystalline form C of eliglustat 1,5-naphthalene disulfonate with X-ray powder diffraction peaks at 2θ angles (± 0.2°) of 9.4°, 13.6°, 20.1°, and 12.1°. The crystalline form C of the eliglustat 1,5-naphthalene disulfonate further comprises X-ray powder diffraction peaks at 2θ angles (± 0.2°) of 24.3°, 12.8°, and 19.6°. The crystalline form C of the eliglustat 1,5-naphthalene disulfonate further comprises X-ray powder diffraction peaks at 2θ angles (± 0.2°) of 6.2°, and 14.0°. The crystalline form C of the eliglustat 1,5-naphthalene disulfonate has an X-ray powder diffraction pattern that is substantially similar to Fig. 5A.

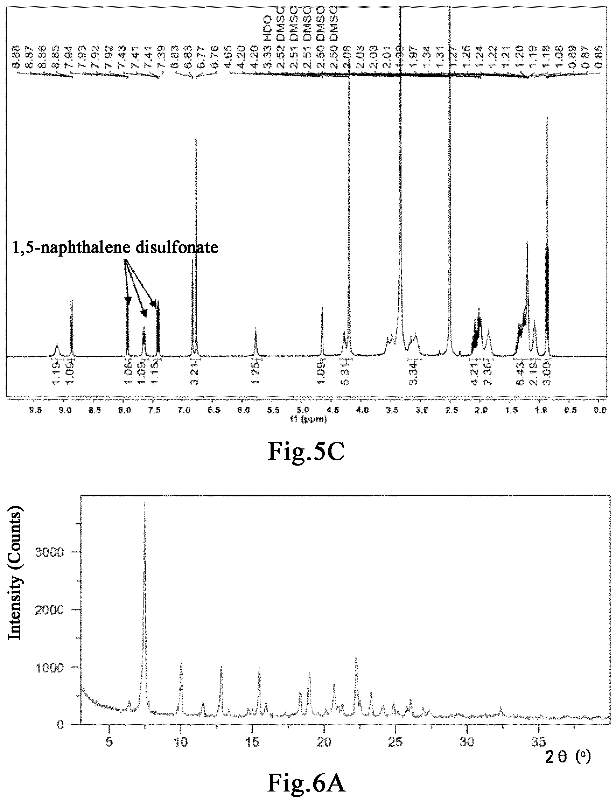

In some embodiments, there is provided a crystalline form A of eliglustat oxalate with X-ray powder diffraction peaks at 2θ angles (± 0.2°) of 7.5°, 15.5°, and 19.0°. The crystalline form A of the eliglustat oxalate further comprises X-ray powder diffraction peaks at 2θ angles (± 0.2°) of 10.0°, 22.3°, and 23.3°. The crystalline form A of the eliglustat oxalate further comprises X-ray powder diffraction peaks at 2θ angles (± 0.2°) of 12.8°, 18.3°, and 20.7°. In some embodiments, the crystalline form A of the eliglustat oxalate has an X-ray powder diffraction pattern that is substantially similar to Fig. 6A.

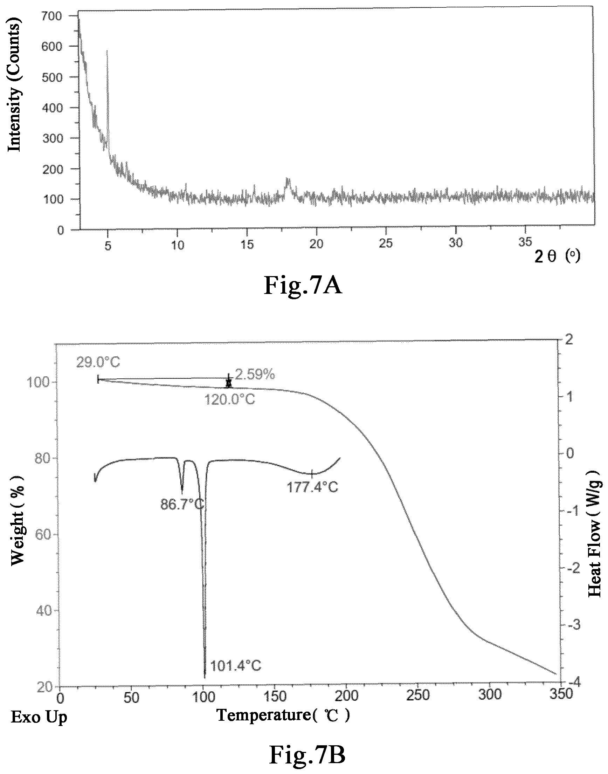

In some embodiments, there is provided a crystalline form A of eliglustat glutarate with X-ray powder diffraction peaks at 2θ angles (± 0.2°) of 5.1°, 19.3°, and 21.3°. The crystalline form A of the eliglustat glutarate further comprises X-ray powder diffraction peaks at 2θ angles (± 0.2°) of 15.5°, 6.4°, and 10.6°. The crystalline form A of the eliglustat glutarate further comprises X-ray powder diffraction peaks at 2θ angles (± 0.2°) of 18.6°, 21.9°, and 13.1°. In some embodiments, the crystalline form A of the eliglustat glutarate has an X-ray powder diffraction pattern that is substantially similar to Fig. 7A.

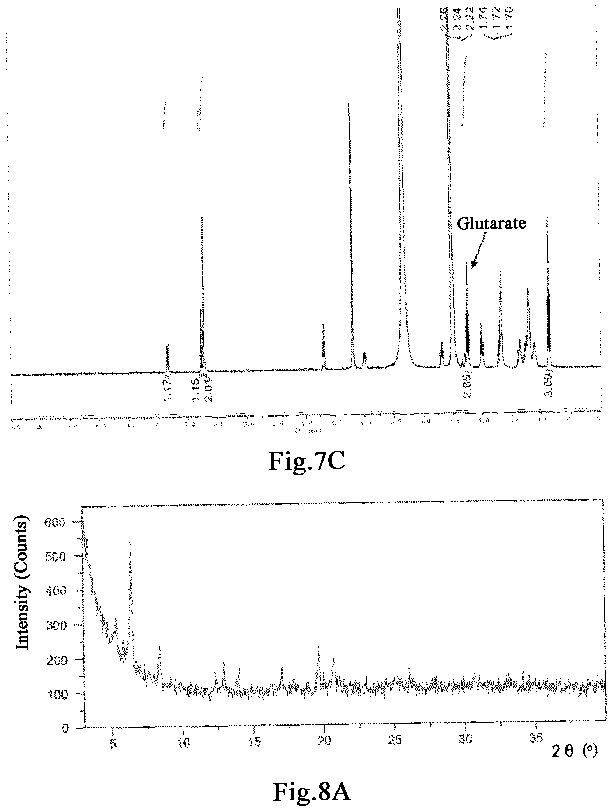

In some embodiments, there is provided a crystalline form A of eliglustat mucate with X-ray powder diffraction peaks at 2θ angles (± 0.2°) of 6.4°, 8.4°, and 20.7°. The crystalline form A of the eliglustat mucate further comprises X-ray powder diffraction peaks at 2θ angles (± 0.2°) of 5.3°, 14.0°, and 12.4°. The crystalline form A of the eliglustat mucate further comprises X-ray powder diffraction peaks at 2θ angles (± 0.2°) of 17.0°, 19.6°, and 17.9°± 0.2°. In some embodiments, the crystalline form A of the eliglustat mucate has an X-ray powder diffraction pattern that is substantially similar to Fig. 8A.

In some embodiments, the pharmaceutically acceptable salt of eliglustat of the present invention, wherein the compound is at least 60% by weight of the monomorph form, at least 70% by weight of the monomorph form, at least 80% by weight of the monomorph form, at least 90% by weight of the monomorph form, at least 95% by weight of the monomorph form, or at least 99% by weight of the monomorph form.

The present invention also provides a pharmaceutical composition, of which the active ingredients comprise pharmaceutically acceptable salts of eliglustat, hydrate of eliglustat 1,5-naphthalene disulfonate, crystalline form D of eliglustat 1,5-naphthalene disulfonate, crystalline form B of eliglustat 1,5-naphthalene disulfonate, crystalline form C of eliglustat 1,5-naphthalene disulfonate, crystalline form A of eliglustat oxalate, crystalline form A of eliglustat glutarate, or crystalline form A of eliglustat mucate, and the pharmaceutically acceptable carrier.

The compositions of the invention can be administered orally, parenterally, by inhalation spray, topically, rectally, nasally, bucally, transmucosally or in ophthalmic formulations. The term "parenterally" as used herein includes subcutaneous, intravenous, intramuscular, intraarticular, intrasynovial, intrasternal, intrathecal, intrahepatic, intralesional, and intracranial injection or infusion techniques. In one aspect, the invention provides pharmaceutical compositions for oral administration in orally acceptable dosage forms, including but not limited to capsules, tablets, emulsions and aqueous suspensions, dispersions and solutions.

A pharmaceutically acceptable carrier refers to a nontoxic carrier, adjuvant or vehicle, which does not adversely impact the pharmacological activity of the compound with which it is formulated, and is safe for human use.

In one embodiment, the pharmaceutical compositions provided herein comprise one or more selected from the group consisting of diluents, disintegrants, binders, surfactants, glidants, and lubricants.

The present invention also provides a method for preparing crystalline form D of eliglustat 1,5-naphthalene disulfonate. In some embodiments, the following steps are comprised: the eliglustat free base and 1-1.1 equivalents of 1,5-naphthalene disulfonic acid are dissolved in methyl tert-butyl ether, the mixture system is magnetically stirred at room temperature and then centrifuged, the obtained solid is dried under vacuum at room temperature overnight to obtain the crystalline form D of eliglustat 1,5-naphthalene disulfonate.

In some embodiments, the preparation method for crystalline form D of eliglustat 1,5-naphthalene disulfonate comprises the following steps: the eliglustat free base and 1-1.1 equivalents of 1,5-naphthalene disulfonic acid are dissolved in methyl tert-butyl ether, the mixture system is magnetically stirred at room temperature for 1-3 days and then centrifuged, the obtained solid is dried under vacuum at room temperature overnight. The 1,5-naphthalene disulfonic acid can be a non-solvate or a hydrate, and specifically, the tetrahydrate of 1,5-naphthalene disulfonic acid is selected.

The present invention also provides a method for preparing crystalline form B of eliglustat 1,5-naphthalene disulfonate. In some embodiments, the following steps are comprised: the eliglustat free base and 0.5 equivalents of 1,5-naphthalene disulfonic acid are dissolved in tetrahydrofuran/n-heptane for continuous suspending and stirring, and the precipitated solid is dried to obtain the crystalline form B of eliglustat 1,5-naphthalene disulfonate.

In some embodiments, the preparation method for crystalline form B of eliglustat 1,5-naphthalene disulfonate comprises the following steps: the eliglustat free base and 0.5 equivalents of 1,5-naphthalene disulfonic acid are dissolved in tetrahydrofuran/n-heptane (1:9, v:v) for continuous suspending and stirring at a temperature of 20-40°C, and the precipitated solid is dried to obtain the crystalline form B of eliglustat 1,5-naphthalene disulfonate.

The present invention provides a method for preparing crystalline form C of eliglustat 1,5-naphthalene disulfonate. In some embodiments, the following steps are comprised: the crystalline form B of eliglustat 1,5-naphthalene disulfonate is added to H₂O, and stirred at room temperature, the solid is separated and dried with calcium oxide to obtain crystalline form C of eliglustat 1,5-naphthalene disulfonate.

In some embodiments, the method for preparing crystalline form C of eliglustat 1,5-naphthalene disulfonate comprises the following steps: the crystalline form B of eliglustat 1,5-naphthalene disulfonate is added to 10 times the weight of H₂O, and stirred at room temperature overnight, the solid is separated and dried in a desiccator filled with calcium oxide for 24 hours to obtain crystalline form C of eliglustat 1,5-naphthalene disulfonate.

The present invention also provides a method for preparing crystalline form A of eliglustat oxalate. In some embodiments, the following steps are comprised: the eliglustat free base and 0.5 equivalents of oxalic acid are dissolved in methyl tert-butyl ether for continuously suspending and stirring at 15-50°C, and the precipitated solid is dried to obtain the crystalline form A of eliglustat oxalate.

In some embodiments, the method for preparing crystalline form A of eliglustat oxalate comprises the following steps: the eliglustat free base is dissolved in methyl tert-butyl ether, and 0.5 equivalents of oxalic acid is added in batches with stirring for continuously suspending and stirring at 40°C. Upon overnight reaction, the solid is separated by suction filtration and dried under vacuum at room temperature to obtain the crystalline form A of eliglustat oxalate.

The present invention also provides a method for preparing crystalline form A of eliglustat glutarate. In some embodiments, the following steps are comprised: the eliglustat free base and 0.5 equivalents of glutaric acid are dissolved in isopropyl acetate/n-heptane (1:5, v:v) for continuously suspending and stirring at 20-40°C, and the precipitated solid is dried to obtain the crystalline form A of eliglustat glutarate.

In some embodiments, the method for preparing crystalline form A of eliglustat glutarate comprises the following steps: the eliglustat free base and 0.5 equivalents of glutaric acid are dissolved in isopropyl acetate/n-heptane (1:5, v:v) for continuously suspending and stirring at room temperature for 1 day and 40°C for 2 days, and the precipitated solid is dried to obtain the crystalline form A of eliglustat glutarate.

The present invention also provides a method for preparing crystalline form A of eliglustat murate. In some embodiments, the following steps are comprised: the eliglustat free base and 0.5 equivalents of mucic acid are dissolved in acetone/n-heptane (1:9, v:v) for continuously suspending and stirring at 35-45°C, and the precipitated solid is dried to obtain the crystalline form A of eliglustat murate.

In some embodiments, the method for preparing crystalline form A of eliglustat mucate comprises the following steps: the eliglustat free base is dispersed in acetone/n-heptane (1:9, v:v), and 0.5 equivalents of mucic acid is added in batches with stirring, the system is circulated at a temperature of 50°C to 5°C for 2-4 times, the solid is separated, washed with n-heptane, and dried under vacuum at room temperature to obtain the crystalline form A of eliglustat murate.

The present invention also provides the use of pharmaceutically acceptable salt of eliglustat, hydrate of eliglustat 1,5-naphthalene disulfonate, crystalline form D of eliglustat 1,5-naphthalene disulfonate, crystalline form B of eliglustat 1,5-naphthalene disulfonate, crystalline form C of eliglustat 1,5-naphthalene disulfonate, crystalline form A of eliglustat oxalate, crystalline form A of eliglustat glutarate, crystalline form A of eliglustat mucate in the treatment of Gaucher's disease, Fabry's disease, and polycystic kidney disease.

The present invention also provides the use of pharmaceutically acceptable salt of eliglustat, hydrate of eliglustat 1,5-naphthalene disulfonate, crystalline form D of eliglustat 1,5-naphthalene disulfonate, crystalline form B of eliglustat 1,5-naphthalene disulfonate, crystalline form C of eliglustat 1,5-naphthalene disulfonate, crystalline form A of eliglustat oxalate, crystalline form A of eliglustat glutarate, crystalline form A of eliglustat mucate in the manufacture of a medicament in treating of Gaucher's disease, Fabry's disease, and polycystic kidney disease.

In some embodiments, the patient with the disease is an extensive, intermediate or poor metabolizer of CYP2D6.

In some embodiments, Gaucher's disease is Type I Gaucher's disease and the polycystic kidney disease is autosomal dominant polycystic kidney disease.

### Definition

When describing a pharmaceutically acceptable salt of eliglustat, the terms "form A", "form B", and "form C" refer to crystalline forms A, B and C of the pharmaceutically acceptable salts of eliglustat, respectively, when used alone. "Form A" and "crystalline forms A", "form B" and "crystalline forms B" and "form C" and "crystalline forms C" are used interchangeably herein.

As used herein, "crystalline" refers to a solid form of eliglustat or a pharmaceutically acceptable salt thereof, wherein long-range atomic ordering of atomic positions is present. The crystalline nature of the solid can be confirmed, for example, by examining X-ray powder diffraction patterns. If the XRPD shows sharp intensity peaks in the XRPD, the compound is crystalline. As "crystalline" are solids that are fully crystalline or partially crystalline, and comprises solids that are at least 80% crystalline, 85% crystalline, 90% crystalline, 95% crystalline, and 99% crystalline by weight.

The term "solvate" refers to a stoichiometric or non-stoichiometric amount of a solvent or solvent mixture introduced into a crystalline structure.

The term "hydrate" refers to a stoichiometric or non-stoichiometric amount of water introduced into a crystalline structure. A hydrate is a solvate wherein the solvent incorporated into the crystalline structure is water. The term "anhydrous" when used with respect to a compound refers that there is substantially no solvent incorporated into the crystalline structure, e.g. less than 0.1% by weight as determined by TGA, Karl-Fisher analysis, monomorph data. Compounds that are anhydrous are referred as "anhydrous" herein.

The term "main peaks" refers to the three strongest peaks in the XRPD data pattern, or to the peak with at least 50% of the 100% relative intensity of the strongest peak.

The specific 2θ values in the XRPD lists of each crystalline form herein retain four decimal places. The numerical value of each data with one, two, three or four decimal places is regarded as the specific data disclosed in the present application and is comprised in the disclosure of the present application. The 2θ values of the X-ray powder diffraction patterns of the crystalline forms described herein may vary slightly from instrument-to-instrument, as well as to differences in sample preparation and batch-to-batch variation. Therefore, unless otherwise specified, XRPD patterns and/or 2θ peaks described herein should not be interpreted as absolute and may vary ±0.2 degrees. The 2θ values presented herein were obtained with Cu Kα1 radiation.

Temperature values (e.g., DSC peak temperature and DSC onset temperature) may vary slightly from instrument-to-instrument and also depend on variations in sample preparation, the rate of temperature rise during the experiment, batch-to-batch variation in materials, and other environmental factors. Therefore, unless otherwise specified, temperature values stated herein should not be interpreted as absolute and may vary ±5°C.

"Substantially identical XRPD patterns" or "substantially similar X-ray powder diffraction patterns" refer that, for comparison purposes, at least 90% of the displayed peaks are present. It should be further understood that for comparison purposes, some variation, such as ±0.2 degrees, between the 2θ peak position and the displayed position is allowed. It should be understood that when the expression "X-ray powder diffraction peaks characterized by a 2θ angle (±0.2°)" is followed by a list of 2θ peak positions of ±0.2°, this applies to each listed peak position.

### BRIEF DESCRIPTION OF THE FIG.S

Fig. 1A is the XRPD pattern of crystalline form A of eliglustat tartrate;
Fig. 1B is the TGA/DSC profile of crystalline form A of eliglustat tartrate;
Fig. 1C is the ¹H HMR spectrum of crystalline form A of eliglustat tartrate;
Fig. 2A is the XRPD pattern of crystalline form A of eliglustat free base;
Fig. 2B is the TGA/DSC profile of crystalline form A of eliglustat free base;
Fig. 2C is the ¹H HMR spectrum of crystalline form A of eliglustat free base;
Fig. 3A is the XRPD pattern of crystalline form D of eliglustat 1,5-naphthalene disulfonate;
Fig. 3B is the TGA/DSC profile of crystalline form D of eliglustat 1,5-naphthalene disulfonate;
Fig. 3C is the ¹H HMR spectrum of crystalline form D of eliglustat 1,5-naphthalene disulfonate;
Fig. 4A is the XRPD pattern of crystalline form B of eliglustat 1,5-naphthalene disulfonate;
Fig. 4B is the TGA/DSC profile of crystalline form B of eliglustat 1,5-naphthalene disulfonate;
Fig. 4C is the ¹H HMR spectrum of crystalline form B of eliglustat 1,5-naphthalene disulfonate;
Fig. 5A is the XRPD pattern of crystalline form C of eliglustat 1,5-naphthalene disulfonate;
Fig. 5B is the TGA/DSC profile of crystalline form C of eliglustat 1,5-naphthalene disulfonate;
Fig. 5C is the ¹H HMR spectrum of crystalline form C of eliglustat 1,5-naphthalene disulfonate;
Fig. 6A is the XRPD pattern of crystalline form A of eliglustat oxalate;
Fig. 6B is the TGA/DSC profile of crystalline form A of eliglustat oxalate;
Fig. 6C is the ¹H HMR spectrum of crystalline form A of eliglustat oxalate;
Fig. 7A is the XRPD pattern of crystalline form A of eliglustat glutarate;
Fig. 7B is the TGA/DSC profile of crystalline form A of eliglustat glutarate;
Fig. 7C is the ¹H HMR spectrum of crystalline form A of eliglustat glutarate;
Fig. 8A is the XRPD pattern of crystalline form A of eliglustat mucate;
Fig. 8B is the TGA/DSC profile of crystalline form A of eliglustat mucate;
Fig. 8C is the ¹H HMR spectrum of crystalline form A of eliglustat mucate.

### DETAILED DESCRIPTION

Crystalline and amorphous forms are prepared following the following general process, as described in the Examples below.

Typical abbreviations of solvent used in the present invention are summarized below:

**Table 1. Abbreviations of Solvent**

| **Abbreviation** | **Full Name** | **Abbreviation** | **Full Name** |
|---|---|---|---|
| MeOH | Methanol | 1,4-Dioxane | 1,4-Dioxane |
| EtOH | Ethanol | ACN | Acetonitrile |
| IPA | Isopropanol | DCM | Dichloromethane |
| Acetone | Acetone | CHCl₃ | Chloroform |
| MIBK | Methyl Isobutyl Ketone | Toluene | Toluene |
| EtOAc | Ethyl Acetate | n-heptane | N-Heptane |
| IPAc | Isopropyl Acetate | DMSO | Dimethyl sulfoxide |
| MTBE | Methyl Tert-Butyl Ether | Anisole | Anisole |
| THF | Tetrahydrofuran | CPME | Cyclopentyl Methyl Ether |
| 2-MeTHF | 2-Methyltetrahydrofuran | H₂O | Water |

### Solution

### Preparation of simulated gastric fluid (SGF)

0.2g sodium chloride and 0.1g Triton X-100 are weighted into a 100 mL volumetric flask. Pure water is added for dissolution, stirred until the solid is completely dissolved, about 1.632 mL hydrochloric acid (1M) is added, and the pH is adjusted to 1.8 with 1M hydrochloric acid or 1M sodium hydroxide, and finally it is diluted to volume with pure water.

### Preparation of fasting state simulating intestinal fluid (FaSSIF)

0.34g sodium dihydrogen phosphate (NaH2PO4, anhydrous), 0.042g sodium hydroxide, and 0.62g sodium chloride are weighted respectively into a 100 mL volumetric flask, about 48 mL pure water is added for dissolution, the pH is adjusted to 6.5 with 1M hydrochloric acid or 1M sodium hydroxide, and finally it is diluted to volume with pure water to obtain a stock solution. A 50-mL volumetric flask is taken. 0.11g of SIF powder is added, and dissolved with the above stock solution, then diluted to volume. The powder is completely dissolve by ultrasound.

### XRPD

XRPD patterns are collected on an X-ray powder diffraction analyzer produced by PANalytacal, and Table 2 lists the XRPD parameters.

**Table 2. XRPD parameters**

| **Parameter** | **Instrument 1** | **Instrument 2** |
|---|---|---|
| Mode | Empyrean | X'pert 3 |

| X-ray wavelength | Cu, kα, Kα1 (Å): 1.540598, Kα2 (Å): 1.544426 Intensity ratio of Kα2/Kα1: 0.50 | Cu, kα, Kα1 (Å): 1.540598, Kα2 (Å): 1.544426 Intensity ratio of Kα2/Kα1: 0.50 |
|---|---|---|
| Settings of X-ray tube | 45 kV, 40 mA | 45 kV, 40 mA |
| Divergent slit | 1/8° | 1/8° |
| Mode of Scanning | continuous | continuous |
| Scope of Scanning (°2θ) | 3-40 | 3-40 |
| Time for each step (s) | 17.8/33.0 | 46.7 |
| Length of step (°2θ) | 0.0167 | 0.0263 |
| Time for test | ~5 min 30'/~10 min 13' | ~5 min |

### TGA and DSC

TGA and DSC profiles are collected on TA Q5000/Discovery 5500 Thermal Gravimetric Analyzer and TA Q2000/Discovery 2500 Differential Scanning Calorimeter, respectively. See Table 3 for detailed parameters.

**Table 3. TGA and DSC parameters**

| **Parameter** | **TGA** | **DSC** |
|---|---|---|
| Method | linear heating | linear heating |
| Sample pan | Aluminum pan, open | Aluminum pan, covered/un-covered |
| Temperature | Room temperature to the set end temperature | 25°C to the set end temperature |
| Heating rate (°C/min) | 10 | 10 |
| Flow gas | N₂ | N₂ |

### Dynamic Vapor Sorption (DVS)

Dynamic Vapor Sorption (DVS) profiles are collected on the DVS Intrinsic of SMS (Surface Measurement Systems). Relative humidity at 25°C is corrected with the deliquescent points of LiCl, Mg(NO₃)₂ and KCl. Table 4 lists the parameters of the DVS test.

**Table 4. DVS parameters**

| **Parameter** | **Set Value** |
|---|---|
| Temperature | 25°C |
| Amount of sample | 10 ∼ 20 mg |
| Shielding gas and flow rate | N₂, 200 mL/min |
| dm/dt | 0.002 %/min |
| Minimum Equilibrium Time for dm/dt | 10 min |
| Maximum Equilibrium Time | 180 min |
| Scope of RH | 0% RH to 95% RH |
| Gradient of RH | 10% (0%RH-90%RH, 90%RH-0%RH) |
| | 5% (90%RH-95%RH, 95%RH-90%RH) |

### Solution NMR

Solution NMR spectra are collected on a Bruker 400M NMR instrument with DMSO-*d₆* as the solvent.

### Ion Chromatography/High Performance Liquid Chromatography (IC/HPLC)

The purity test, dynamic solubility and stability test in the experiment are completed by Agilent 1260 high performance liquid chromatography. The salt-forming molar ratio test of ions is completed by ion chromatography test. The analysis conditions are as shown in Table 5 and Table 6.

**Table 5. Test Conditions for High Performance Liquid Chromatography**

| | | |
|---|---|---|
| HPLC | Agilent 1260 DAD Detector | |
| Column | Xbridge C18, 4.6 × 150 nm, 3.5 µm | |
| Mobile Phase | A: 0.02mol/L KH₂PO₄ in H₂O (0.1% TEA) | |

| | B: ACN | |
|---|---|---|
| Gradient Table | Time (min) | %B |
| | 0.0 | 20 |
| | 7.0 | 90 |
| | 8.0 | 90 |
| | 8.1 | 20 |
| | 10.0 | 20 |
| | 0.0 | 20 |
| Operation Time | 10.0 min | |
| Flow Rate | 1.0 mL/min | |
| Injection Volume | 5µL | |
| Detector Wavelength | UV at 202 nm | |
| Temperature of Column | 30°C | |
| Temperature of Injector | RT | |
| Diluent | ACN/H₂O (1:1, v/v) | |

**Table 6. Test Conditions for Ion Chromatography**

| **Ion Chromatograph** | **ThermoFisher ICS-1100** |
|---|---|
| Column | IonPac AS 18 Analytical Column, 250*4 mm |
| Mobile Phase | 25 mM NaOH |
| Injection Volume | 25 µL |
| Flow Rate | 1.0 mL/min |
| Temperature | 35°C |
| Temperature of Column | 35°C |
| Current | 80 mA |
| Operation Time | Cl⁻: 7.0 min, C₂O₄²⁻: 6.0 min, PO₄³⁻: 40.0 min |

### Example 1 Preparation and characterization of crystalline form A of eliglustat tartrate.

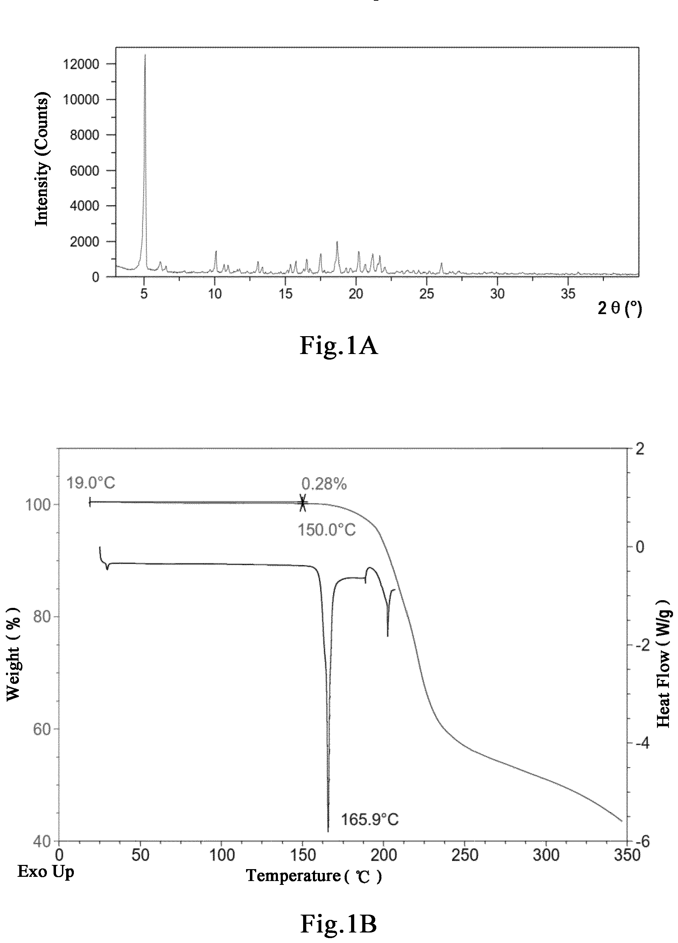

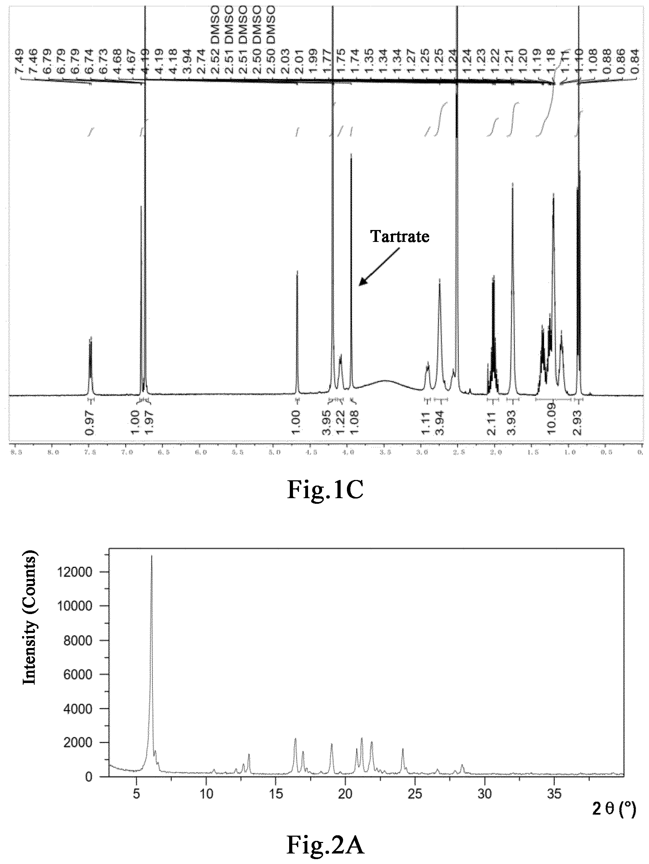

Eliglustat tartrate was obtained from the market, and the XRPD pattern thereof was measured. The XRPD pattern of the eliglustat tartrate is shown in Fig. 1A, which shows that the sample is in a crystalline from, and consistent with the XRPD pattern data in Fig. 1 of the specification in WO2011066325A1. It is described as the crystalline form A of eliglustat tartrate of the present patent. The TGA/DSC results (Fig. 1B) show that the sample has a weight loss of 0.2% when heated to 150°C, and an endothermic peak is observed at 165.9°C (peak temperature). ¹H NMR was measured in DMSO-*d₆*. The results are shown in Fig. 1C. The results show that the molar ratio of tartaric acid to API is about 0.5, and no obvious organic solvent residue is detected.

### Example 2 Preparation and characterization of free eliglustat

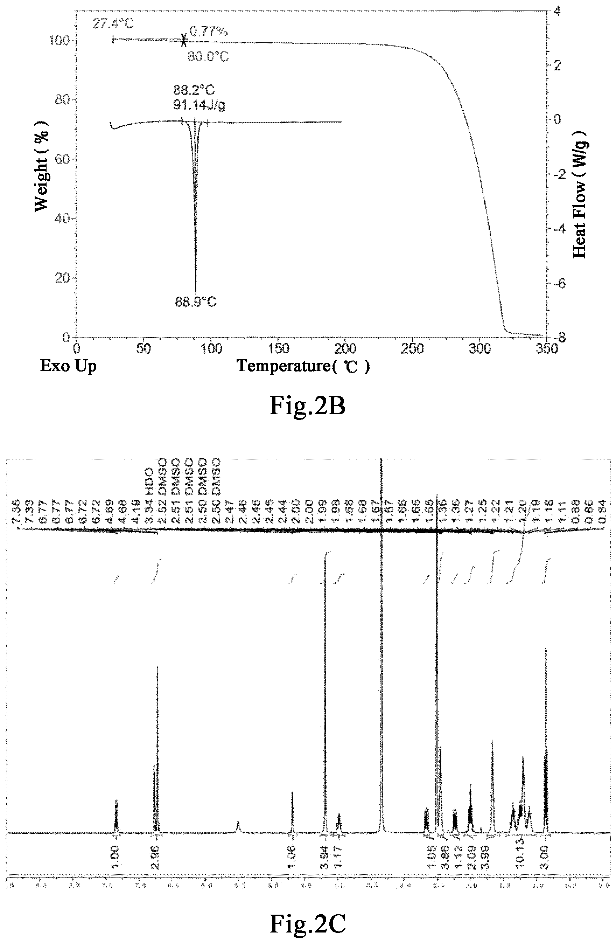

The free crystalline from A was obtained from the following method: about 200 mg of the crystalline form A of initial tartrate was weighted in a beaker, 2 mL of deionized water was added for dissolution, 20 mL of saturated NaHCO₃ solution was quickly added to the solution, and stirred at room temperature for 1.5 hours. The obtained solid was collected by suction filtration, and upon vacuum drying at room temperature overnight, the XRPD of the obtained sample was tested. Fig. 2A shows that the sample is in a crystalline form, named as free crystalline from A. The XRPD result thereof is shown in Fig. 2A. The TGA/DSC results (Fig. 2B) show that the sample has a weight loss of 0.8% when heated to 80°C, and an endothermic peak is observed at 88.9°C (peak temperature). ¹H NMR was measured in DMSO-*d₆*. The results are shown in Fig. 2C. No obvious organic solvent residue is detected.

### Example 3 Screening for salt form

The prepared free crystalline from A was used as raw material, 28 acidic ligands were selected, and a total of 154 salt form screening tests were set up in three stages. The specific steps of the screening test were as follows: about 20 mg of the free crystalline from A sample and the corresponding ligand were weighted into an HPLC vial, 0.5 mL of solvent was added and mixed to obtain a suspension. Upon suspending and stirring at room temperature for 2 days (the third round screening test was conducted at 40°C), the solid was separated by centrifugation and dried under vacuum at room temperature. After stirring at room temperature, the obtained clear solution was transferred to 5°C for stirring or an antisolvent (n-heptane) was added to induce the precipitation of a solid. If it was still clear, the solution was transferred to room temperature for open volatilization to obtain a solid; the obtained Colloidal substance was transferred to a temperature cycle of 50°C to 5°C (one cycle: heating to 50°C at the heating rate of 4.5°C/min, keep temperature at 50°C for 2 hours; cooling to 5°C at the cooling rate of 0.1°C/min; keep temperature at 5°C for 2 hours). The obtained solids were tested for XRPD, and the results showed that a total of 11 salt forms were obtained in the three-stage XRPD screening test, including crystalline form A or B of 1,5-naphthalene disulfonate, crystalline form A of oxalate, crystalline form A of mucate, crystalline form A or B of malate, crystalline form A of glutarate, crystalline form A of succinate, crystalline form A of phosphate, crystalline form A of hydrochloride, and crystalline form A of fumarate. The 11 salt crystalline forms were characterized by XRPD, TGA, DSC and ¹H NMR or IC/HPLC. The characterization results are summarized in Table 7-1 to Table 7-4.

**Table 7-1 Summary of the results of the first round screening tests of salt form**

| **Ligand acid** | **Mole ratio of feed (ligand/API)** | **EtOH/ n-heptane (1:1, v:v) (A)** | **MTBE (B)** | **EtOAc (C)** |
|---|---|---|---|---|
| Hydrochloric acid | 1:1 | Colloidal substance | Crystalline form A of hydrochloride | Crystalline form A of hydrochloride |
| Sulfuric acid | 1:1 | Colloidal substance | Colloidal substance | Colloidal substance |
| L-aspartic acid | 1:1 | L-aspartic acid¹ | L-aspartic acid ¹ | L-aspartic acid ¹ |
| Maleic acid | 1:1 | Colloidal substance | Colloidal substance | Colloidal substance |
| Maleic acid | 1:2 | Colloidal substance | Colloidal substance | Colloidal substance |
| Phosphoric acid | 1:1 | Crystalline form A of phosphate² | Crystalline form A of phosphate | Crystalline form A of phosphate |
| Ascorbic acid | 1:1 | Amorphous⁴ | Free crystalline from A¹ | Colloidal substance |
| Fumaric acid | 1:1 | Colloidal substance | Amorphous* | Amorphous * |
| Fumaric acid | 1:2 | Crystalline form A of fumarate² | Amorphous ³ | Amorphous ³ |
| Citric acid | 1:1 | Colloidal substance | Amorphous ¹ | Amorphous ³ |
| Citric acid | 1:2 | Colloidal | Amorphous⁴ | Amorphous³ |
| | | substance | | |
| Citric acid | 1:3 | Colloidal substance | Amorphous ³ | Amorphous ³ |
| D-glucuronic acid | 1:1 | Amorphous | Weak crystallinity* | Weak crystallinity* |
| L-malic acid | 1:1 | Colloidal substance | Colloidal substance | Crystalline form A of malate * |
| L-malic acid | 1:2 | Colloidal substance | Amorphous | Colloidal substance |
| Hippuric acid | 1:1 | Colloidal substance | Amorphous | Colloidal substance |
| D-gluconic acid | 1:1 | Colloidal substance | Amorphous 3 | Colloidal substance |
| Lactic acid | 1:1 | Colloidal substance | Colloidal substance | Colloidal substance |
| Succinic acid | 1:1 | Colloidal substance | Colloidal substance | Colloidal substance |
| Succinic acid | 1:2 | Crystalline form A of succinate ⁵ | Colloidal substance | Colloidal substance |
| Adipic acid | 1:1 | Colloidal substance | Colloidal substance | Colloidal substance |
| Acetic acid | 1:2 | Colloidal substance | Colloidal substance | Colloidal substance |
| Ethylene disulfonic acid | 1:1 | Colloidal substance | Amorphous | Amorphous ³ |
| 1,5-naphthalene disulfonic acid | 1:1 | Colloidal substance | Crystalline form A of 1,5-naphthalene disulfonate | Weak crystallinity |
| P-toluenesulfonic acid | 1:1 | Colloidal substance | Colloidal substance | Colloidal substance |
| Methanesulfonic acid | 1:1 | Colloidal substance | Colloidal substance | Colloidal substance |
| Benzenesulfonic acid | 1:1 | Colloidal substance | Colloidal substance | Colloidal substance |
| Oxalic acid | 1:1 | Crystalline form A of oxalate + additional peaks ¹ | Crystalline form A of oxalate | Crystalline form A of oxalate |
| Oxalic acid | 1:2 | Crystalline form A of oxalate ¹ | Crystalline form A of oxalate | Crystalline form A of oxalate + additional peaks |
| Hydroxyethanesulfonic acid | 1:1 | Colloidal substance | Colloidal substance | Colloidal substance |
| Propanedioic acid | 1:1 | Colloidal substance | Colloidal substance | Colloidal substance |
| Gentisic acid | 1:1 | Colloidal substance | Weak crystallinity | Amorphous ² |
| Benzoic acid | 1:1 | Colloidal substance | Colloidal substance | Colloidal substance |

| | | | | |
|---|---|---|---|---|
| *: The solid deliquesces during XRPD test; Colloidal substance: The sample is still a colloidal substance upon low-temperature stirring, anti-solvent addition, temperature cycling, and exposure evaporation at room temperature; 1: It was stirred at room temperature to obtain a clear solution. It was stirred at 5°C and -20°C to obtain a solid for XRPD testing; 2: It was stirred at room temperature and 5°C to obtain a clear solution. N-heptane was added as an antisolvent to obtain a colloidal substance. Upon temperature cycling (50°C to 5°C), a solid was obtained for XRPD testing; 3: It was stirred at room temperature to obtain a colloidal substance. Upon temperature cycling (50°C to 5°C), a solid was obtained for XRPD testing; 4: It was stirred at room temperature to obtain a clear solution. It was stirred at 5°C to obtain a colloidal substance. Upon temperature cycling (50°C to 5°C), a solid was obtained for XRPD testing; 5: It was stirred at room temperature to obtain a clear solution. N-heptane was added as an antisolvent to obtain an oily substance. Upon exposure evaporation at room temperature, a solid was obtained for XRPD testing. | | | | |

**Table 7-2 Summary of the results of the second round screening tests of salt form**

| **Ligand acid** | **Mole ratio of feed (ligand/API)** | **MeOH/Acetone (5:95, v:v)** | **H₂O/Acetone (1:99, v:v)** |
|---|---|---|---|
| Maleic acid | 1:2 | Clear solution | Clear solution |
| Fumaric acid | 1:2 | Clear solution | Clear solution |
| Succinic acid | 1:2 | Clear solution | Clear solution |
| L-malic acid | 1:2 | Clear solution | Clear solution |
| Mucic acid | 1:2 | Crystalline of mucic acid | Crystalline of mucic acid |

| | | | |
|---|---|---|---|
| Clear solution: No crystalline was observed. | | | |

**Table 7-3 Summary of the results of the third round screening tests of salt form***

| **Ligand acid** | **IPA/n-Heptane (1:12, v:v) (A)** | **Acetone/n-Heptane (1:9, v:v) (B)** | **IPAc/n-Heptane (1:5, v:v) (C)** | **THF/n-Heptane (1:9, v:v) (D)** |
|---|---|---|---|---|
| 1,5-Naphthalene disulfonic acid | Crystalline form B of 1,5-naphthalene disulfonate | Crystalline form B of 1,5-naphthalene disulfonate | Crystalline form B of 1,5-naphthalene disulfonate | Crystalline form B of 1,5-naphthalene disulfonate |
| Oxalic acid | Crystalline form A of oxalate | Crystalline form A of oxalate | Crystalline form A of oxalate ² | Crystalline form A of oxalate |
| L-malic acid | Colloidal substance | Colloidal substance | Crystalline form B of malate + Free crystalline from A | Free crystalline from A¹ |
| Fumaric acid | Colloidal substance | Colloidal substance | Free crystalline from A¹ | Free crystalline from A ² |
| Succinic acid | Colloidal substance | Crystalline form A of succinate ¹ | Crystalline form A of succinate ² | Crystalline form A of succinate ² |
| Maleic acid | Colloidal substance | Amorphous ¹ | Free crystalline from A² | Free crystalline from A |
| Mucic acid | Crystalline form A of mucate | Crystalline form A of mucate | Free crystalline from A² | Crystalline form A of mucate ² |
| Pamoic acid | Pamoic acid | Pamoic acid + Free crystalline from A ¹ | Free crystalline from A² | Pamoic acid |
| Glutaric acid | Amorphous¹ | Crystalline form A of glutarate + Free crystalline from A | Crystalline form A of glutarate + Free crystalline from A | Free crystalline from A |
| Propanedioic acid | Colloidal substance | Colloidal substance | Free crystalline from A | Free crystalline from A |
| Methanesulfonic acid | NA | NA | NA | Colloidal substance |
| p-toluenesulfonic acid | NA | NA | NA | Colloidal substance |

| | | | | |
|---|---|---|---|---|
| *: The mole ratio of feed of this round screening test is 1:2 (ligand acid/API); NA: Not set. 1: After stirring at 40°C, a colloidal substance was obtained. Upon temperature cycling (50°C to 5°C), a solid was obtained for XRPD testing. 2: The initial XRPD test of the sample yielded free crystalline form A. 0.25 eq of the corresponding ligand acid was added and continue stirred at 40°C to obtain a solid for testing. | | | | |

**Table 7-4 Summary of characterization results of salt forms samples obtained from screening**

| **Salt form and crystalline form** | **TGA weight loss (%)** | **DSC endothermic peak °C, peak temperature** | **Solvent residue wt%** | **Mole ratio of salt Ligand: free salt** |
|---|---|---|---|---|
| Crystalline form A of tartrate | 0.2 (to150°C) | 165.9°C | No | 0.5 |
| Free crystalline form A | 0.8% FaSSIF | 88.9 °C | No | |
| Crystalline form A of phosphate | 4.4 (to 100 °C) | 79.7, 106.4, 131.5 | No | 1.4 |
| Crystalline form A of 1,5-naphthalene disulfonate⁴ | 5.8 (to 80 °C) 4.9 (80°C to 150 °C) | 106.3, 135.0 | (MTBE) 1.0 | 1.0 |
| Crystalline form B of 1,5-naphthalene disulfonate | 1.5 (to 150 °C) | 75.9, 167.9 | No | 0.5 |
| Crystalline form C of 1,5-naphthalene disulfonate | 3.1 (to 150 °C) | 53.1, 81.4, 169.3 | No | 0.5 |
| Crystalline form A of oxalate | 7.5 (to 100 °C) | 85.8 | No | No |
| Crystalline form A of malate | | -- | -- | -- |
| Crystalline form B of malate # | 3.7 (to 150 °C) | 87.8, 103.5, 175.6 | No | 0.6 |
| Crystalline form A of hydrochloride | 11.7 (to 150 °C) | 48.6, 93.0 | NA | 1.0 |
| Crystalline form A of fumarate | 9.1 (to 100 °C) | 54.6, 86.8, 134.1 | No | 0.5 |
| Crystalline form A of mucate | 1.9 (to 120 °C) | 104.7, 147.8 | No | 0.5 |
| Crystalline form A of glutarate# | 2.6 (to 150 °C) | 86.7, 101.4, 177.4 | No | 0.7 |
| Crystalline form A of succinate* | 7.3 (to 120 °C) | 83.7, 169.9 | No | 0.6 |

| | | | | |
|---|---|---|---|---|
| *: Deliquescence was observed in the sample during XRPD testing; #: These two salt form samples are a mixture of salt form and free salt samples. --: Because the sample was obviously deliquescent, these characterization data was not collected; NA: Because the sample obtained from screening was insufficient, this characterization was not performed. +: Crystalline form A of 1,5-naphthalene disulfonate was confirmed to be a mixture in subsequent studies, and stable crystalline form D of 1,5-naphthalene disulfonate was obtained in the crystalline form screening. | | | | |

### Screening of crystalline form

### Example 4 Preparation and characterization of crystalline form D of 1,5-naphthalene disulfonate

49.6 mg of Eliglustat free base and 45.7 mg of 1,5-naphthalene disulfonic acid tetrahydrate (1.1 equivalent) were dissolved in 2.0 mL of MTBE. The mixture system was magnetically stirred (about 750 rpm) at room temperature for about 3 days and then centrifuged (10000 rpm, 2 min). The obtained solid was dried under vacuum at room temperature overnight to obtain the crystalline form D of eliglustat 1,5-naphthalene disulfonate.

The XRPD results of samples are shown in Fig. 3A. The TGA/DSC profile is shown in Fig. 3B. The results show that the weight loss when heated to 130°C is 9.82%, and endothermic signals are observed at 114.7 and 159.8 °C (peak temperature). The ¹H NMR spectrum was obtained from DMSO-d₆ test, and is listed in Fig. 3C. The results show that the mole ratio of ligand acid to API is approximately 1.0.

**Table 8 XRPD peak list of crystalline form D of eliglustat 1,5-naphthalene disulfonate**

| **Position[°2θ]** | **Height [CTS]** | **d-value [Å]** | **Relative Intensity [%]** |
|---|---|---|---|
| 3.0941 | 1380.27 | 28.56 | 100.00 |
| 3.5078 | 1133.94 | 25.19 | 82.15 |
| 4.8984 | 980.81 | 18.04 | 71.06 |
| 5.9174 | 667.55 | 14.94 | 48.36 |
| 6.1540 | 661.73 | 14.36 | 47.94 |
| 7.0360 | 452.99 | 12.56 | 32.82 |
| 8.6403 | 250.84 | 10.23 | 18.17 |
| 10.3686 | 470.99 | 8.53 | 34.12 |
| 12.0968 | 270.92 | 7.32 | 19.63 |
| 12.3742 | 329.32 | 7.15 | 23.86 |
| 13.5541 | 215.98 | 6.53 | 15.65 |
| 14.2473 | 295.83 | 6.22 | 21.43 |
| 14.8066 | 402.67 | 5.98 | 29.17 |
| 15.1403 | 795.50 | 5.85 | 57.63 |
| 16.2174 | 276.09 | 5.47 | 20.00 |
| 17.5035 | 242.35 | 5.07 | 17.56 |
| 18.0000 | 228.30 | 4.93 | 16.54 |
| 18.6809 | 345.72 | 4.75 | 25.05 |
| 19.2442 | 204.08 | 4.61 | 14.79 |
| 20.0986 | 293.28 | 4.42 | 21.25 |
| 21.3602 | 264.56 | 4.16 | 19.17 |
| 21.9766 | 267.90 | 4.04 | 19.41 |
| 23.2243 | 302.34 | 3.83 | 21.90 |
| 24.2819 | 309.64 | 3.67 | 22.43 |
| 24.7180 | 320.35 | 3.60 | 23.21 |
| 25.1042 | 229.22 | 3.55 | 16.61 |
| 25.5802 | 174.15 | 3.48 | 12.62 |
| 26.4982 | 158.66 | 3.36 | 11.50 |
| 28.2539 | 108.95 | 3.16 | 7.89 |
| 30.8579 | 31.30 | 2.90 | 2.27 |
| 36.2520 | 18.84 | 2.48 | 1.36 |
| 37.7991 | 22.49 | 2.38 | 1.63 |

### Example 5 Preparation and characterization of crystalline form B of 1,5-naphthalene disulfonate

Free crystalline form A and 0.5 equivalent of 1,5-naphthalene disulfonic acid were suspended and stirred in THF/n-heptane (1:9, v:v) at room temperature for 1 day and at 40°C for 2 days, the solid was separated by centrifugation and dried under vacuum to obtain free crystalline form A.

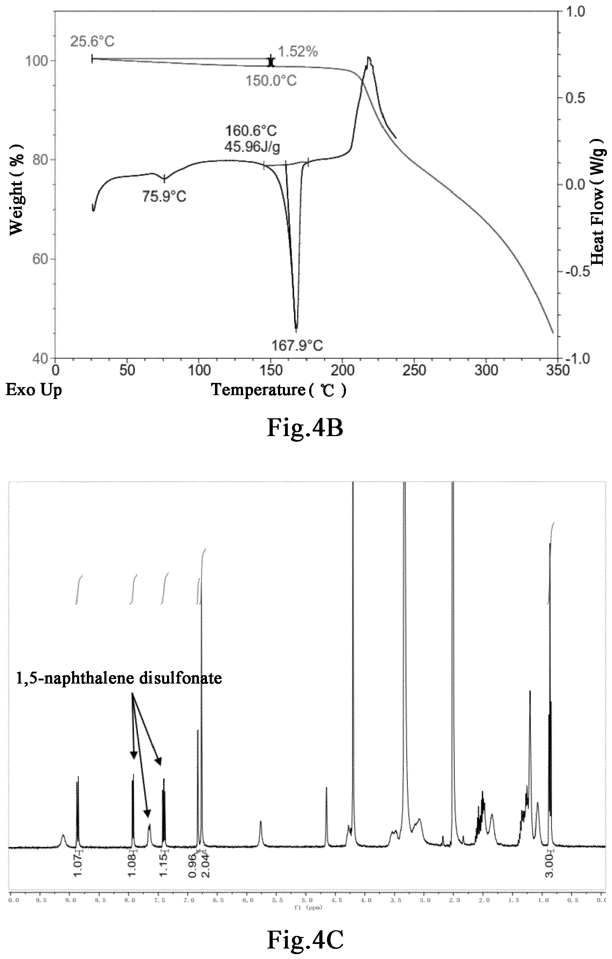

The XRPD results of samples are shown in Fig. 4A. The TGA/DSC profile is shown in Fig. 4B. The results show that the weight loss when heated to 150°C is 1.5%, and endothermic signals are observed at 75.9 and 167.9 °C (peak temperature). The ¹H NMR spectrum was obtained from DMSO-d₆ test, and is listed in Fig. 4C. The results show that the mole ratio of ligand acid to API is approximately 0.5.

**Table 9 XRPD peak list of crystalline form B of eliglustat 1,5-naphthalene disulfonate**

| **Position[°2θ]** | **Height [CTS]** | **d-value [Å]** | **Relative Intensity [%]** |
|---|---|---|---|
| 3.2878 | 1447.58 | 26.87 | 48.57 |
| 6.5266 | 1421.30 | 13.54 | 47.69 |
| 7.2583 | 2980.47 | 12.18 | 100.00 |
| 8.3788 | 126.60 | 10.55 | 4.25 |
| 9.8375 | 106.37 | 8.99 | 3.57 |
| 10.9314 | 411.34 | 8.09 | 13.80 |
| 11.8663 | 109.82 | 7.46 | 3.68 |
| 12.4316 | 141.25 | 7.12 | 4.74 |
| 13.1131 | 831.52 | 6.75 | 27.90 |
| 14.5698 | 2725.75 | 6.08 | 91.45 |
| 15.0911 | 1338.92 | 5.87 | 44.92 |
| 15.9237 | 378.93 | 5.57 | 12.71 |
| 17.0140 | 669.51 | 5.21 | 22.46 |
| 17.4500 | 260.27 | 5.08 | 8.73 |
| 17.8532 | 161.19 | 4.97 | 5.41 |
| 18.2924 | 271.94 | 4.85 | 9.12 |
| 18.8161 | 648.95 | 4.72 | 21.77 |
| 20.8273 | 1127.54 | 4.27 | 37.83 |
| 21.0267 | 1175.33 | 4.23 | 39.43 |
| 21.6603 | 961.20 | 4.10 | 32.25 |
| 21.9779 | 754.23 | 4.04 | 25.31 |
| 22.7865 | 1403.04 | 3.90 | 47.07 |
| 23.1751 | 695.13 | 3.84 | 23.32 |
| 23.9963 | 272.61 | 3.71 | 9.15 |
| 24.3582 | 543.85 | 3.65 | 18.25 |
| 24.9824 | 217.34 | 3.56 | 7.29 |
| 25.3181 | 286.99 | 3.52 | 9.63 |
| 26.2888 | 192.08 | 3.39 | 6.44 |
| 27.5208 | 204.06 | 3.24 | 6.85 |
| 28.3687 | 190.80 | 3.15 | 6.40 |
| 29.2446 | 63.38 | 3.05 | 2.13 |
| 30.3164 | 50.48 | 2.95 | 1.69 |
| 33.0338 | 59.81 | 2.71 | 2.01 |
| 34.7586 | 52.30 | 2.58 | 1.75 |
| 36.6055 | 73.45 | 2.45 | 2.46 |
| 37.5709 | 52.70 | 2.39 | 1.77 |

### Example 6 Preparation and characterization of crystalline form C of 1,5-naphthalene disulfonate

119.9 mg of crystalline form B sample of 1,5-naphthalene disulfonate was weighted into a 3 mL glass bottle, and 2 mL water was added for being stirred magnetically (~750 rpm) at room temperature overnight. The solids were separated by centrifugation and dried in a desiccator containing calcium oxide for 24 hours. The solids were collected for characterization testing and research.

The XRPD results of samples are shown in Fig. 5A. The TGA/DSC profile is shown in Fig. 5B. The results show that the weight loss when heated to 150°C is 6.6%, and endothermic signals are observed at 73.5 and 171.4 °C (peak temperature). The ¹H NMR spectrum was obtained from DMSO-d₆ test, and is listed in Fig. 5C. The results show that the mole ratio of ligand acid to API is approximately 0.5. No obvious THF and n-heptane residues were detected. Identification of crystalline form C of 1,5-naphthalene disulfonate Form C could be conducted by heating tests. Crystalline form C of 1,5-naphthalene disulfonate was converted to crystalline form B of 1,5-naphthalene disulfonate after being heated to 100°C under nitrogen protection and cooled to room temperature. In combination with the results of weight loss of the TGA profile, liquid NMR and results of heating test, crystalline form C of 1,5-naphthalene disulfonate was a dihydrate.

**Table 10 XRPD peak list of crystalline form C of eliglustat 1,5-naphthalene disulfonate**

| **Position[°2θ]** | **Height [CTS]** | **d-value [Å]** | **Relative Intensity [%]** |
|---|---|---|---|
| 3.1524 | 2641.80 | 28.03 | 53.35 |
| 6.2573 | 1068.89 | 14.13 | 21.59 |
| 9.3992 | 717.11 | 9.41 | 14.48 |
| 12.0709 | 3481.21 | 7.33 | 70.30 |
| 12.5525 | 2378.23 | 7.05 | 48.03 |
| 12.7919 | 1399.56 | 6.92 | 28.26 |
| 13.5868 | 2746.15 | 6.52 | 55.46 |
| 14.0051 | 1048.33 | 6.32 | 21.17 |
| 14.8034 | 1805.13 | 5.98 | 36.45 |
| 15.2143 | 1722.90 | 5.82 | 34.79 |
| 15.7903 | 1530.21 | 5.61 | 30.90 |
| 17.0130 | 490.54 | 5.21 | 9.91 |
| 17.2700 | 2086.62 | 5.13 | 42.14 |
| 17.5187 | 1500.41 | 5.06 | 30.30 |
| 17.8955 | 1094.79 | 4.96 | 22.11 |
| 18.0750 | 680.60 | 4.91 | 13.74 |
| 18.5649 | 939.41 | 4.78 | 18.97 |
| 18.8914 | 4951.66 | 4.70 | 100.00 |
| 19.1941 | 295.35 | 4.62 | 5.96 |
| 19.6323 | 1262.85 | 4.52 | 25.50 |
| 20.0970 | 2561.28 | 4.42 | 51.73 |
| 20.3641 | 1304.22 | 4.36 | 26.34 |
| 20.5421 | 2109.61 | 4.32 | 42.60 |
| 21.3901 | 734.11 | 4.15 | 14.83 |
| 21.8198 | 1815.63 | 4.07 | 36.67 |
| 21.9749 | 1058.39 | 4.04 | 21.37 |
| 22.3301 | 206.53 | 3.98 | 4.17 |
| 23.1958 | 1177.05 | 3.83 | 23.77 |
| 23.8812 | 435.90 | 3.73 | 8.80 |
| 24.2840 | 2875.10 | 3.67 | 58.06 |
| 24.6893 | 2288.14 | 3.61 | 46.21 |
| 25.0283 | 608.44 | 3.56 | 12.29 |
| 25.2758 | 733.76 | 3.52 | 14.82 |
| 25.4845 | 1289.12 | 3.50 | 26.03 |
| 25.7603 | 769.45 | 3.46 | 15.54 |
| 26.1552 | 1158.98 | 3.41 | 23.41 |
| 26.4893 | 442.08 | 3.36 | 8.93 |
| 26.9345 | 796.38 | 3.31 | 16.08 |
| 27.3301 | 945.93 | 3.26 | 19.10 |
| 27.6484 | 330.42 | 3.23 | 6.67 |
| 27.9963 | 877.72 | 3.19 | 17.73 |
| 28.4954 | 598.56 | 3.13 | 12.09 |
| 28.6208 | 609.25 | 3.12 | 12.30 |
| 28.8574 | 435.69 | 3.09 | 8.80 |
| 29.5105 | 92.82 | 3.03 | 1.87 |
| 29.9237 | 237.05 | 2.99 | 4.79 |
| 30.2436 | 275.74 | 2.96 | 5.57 |
| 30.7075 | 375.33 | 2.91 | 7.58 |
| 31.2498 | 221.70 | 2.86 | 4.48 |
| 31.8160 | 295.08 | 2.81 | 5.96 |
| 33.8482 | 80.95 | 2.65 | 1.63 |
| 35.0454 | 78.86 | 2.56 | 1.59 |
| 36.2682 | 147.32 | 2.48 | 2.98 |
| 36.7824 | 247.39 | 2.44 | 5.00 |
| 37.6901 | 254.67 | 2.39 | 5.14 |
| 38.0381 | 200.68 | 2.37 | 4.05 |
| 39.1112 | 245.29 | 2.30 | 4.95 |

### Example 7 Preparation and characterization of crystalline form A of oxalate

300.4 mg of free crystalline form A sample was weighted into a 20 mL glass bottle, 15 mL of MTBE was added to dissolve. 33.0 mg of oxalic acid (approximately 0.5 equivalent) was added in batches under magnetic stirring (~750 rpm), and was placed at 40 °C for suspending and stirring. Upon reacting overnight, the solid was separated by suction filtration, dried under vacuum at room temperature, and collected for characterization testing and research.

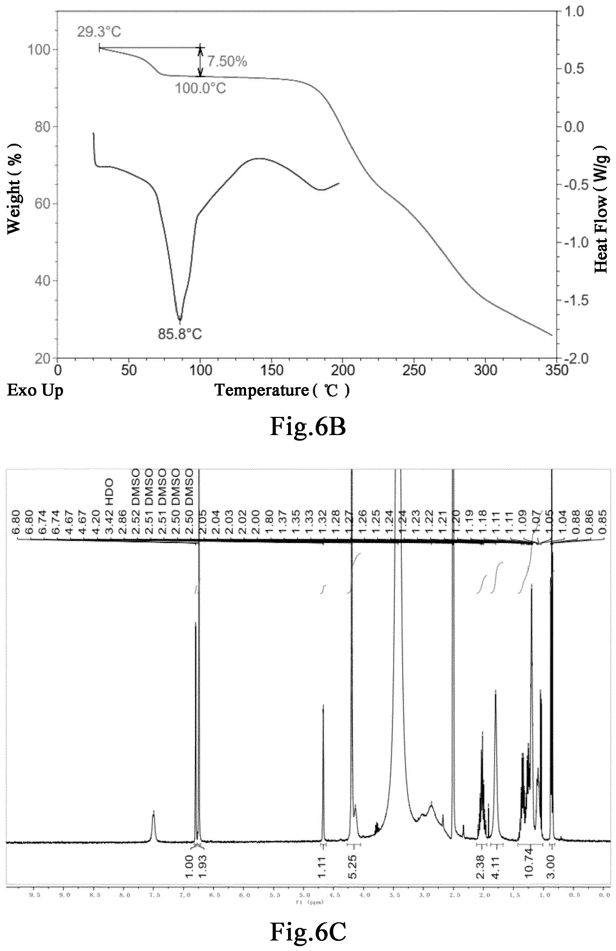

The XRPD results of samples are shown in Fig. 6A. The TGA/DSC profile is shown in Fig. 6B. The results show that the weight loss when heated to 150°C is 7.4%, and endothermic signals are observed at 90.1 and 116.0 °C (peak temperature). The ¹H NMR spectrum was obtained from DMSO-d₆ test, and is listed in Fig. 6C. No obvious MTBE residues in the sample was detected. The results show that the mole ratio of ligand acid to API is approximately 0.5.

**Table 11 XRPD peak list of crystalline form A of eliglustat oxalate**

| **Position[°2θ]** | **Height [CTS]** | **d-value [Å]** | **Relative Intensity [%]** |
|---|---|---|---|
| 3.0796 | 285.61 | 28.69 | 8.03 |
| 6.3983 | 166.08 | 13.81 | 4.67 |
| 7.4721 | 3555.11 | 11.83 | 100.00 |
| 10.0269 | 883.37 | 8.82 | 24.85 |
| 11.5693 | 246.79 | 7.65 | 6.94 |
| 12.8018 | 843.62 | 6.92 | 23.73 |
| 13.3807 | 102.00 | 6.62 | 2.87 |
| 14.6982 | 126.91 | 6.03 | 3.57 |
| 14.9536 | 140.37 | 5.92 | 3.95 |
| 15.4689 | 831.31 | 5.73 | 23.38 |
| 15.9249 | 233.57 | 5.57 | 6.57 |
| 17.2850 | 76.36 | 5.13 | 2.15 |
| 18.3444 | 451.19 | 4.84 | 12.69 |
| 18.9603 | 769.66 | 4.68 | 21.65 |
| 19.5635 | 71.79 | 4.54 | 2.02 |
| 20.1348 | 125.71 | 4.41 | 3.54 |
| 20.6710 | 511.12 | 4.30 | 14.38 |
| 21.2874 | 228.53 | 4.17 | 6.43 |
| 22.2511 | 1031.63 | 4.00 | 29.02 |
| 22.5302 | 261.28 | 3.95 | 7.35 |
| 23.2834 | 404.97 | 3.82 | 11.39 |
| 24.1549 | 207.34 | 3.68 | 5.83 |
| 24.8564 | 245.10 | 3.58 | 6.89 |
| 25.7773 | 208.01 | 3.46 | 5.85 |
| 26.0578 | 299.40 | 3.42 | 8.42 |
| 26.9154 | 167.47 | 3.31 | 4.71 |
| 27.3201 | 129.33 | 3.26 | 3.64 |
| 28.8469 | 47.18 | 3.10 | 1.33 |
| 29.3743 | 56.05 | 3.04 | 1.58 |
| 32.3410 | 179.12 | 2.77 | 5.04 |
| 35.1699 | 53.48 | 2.55 | 1.50 |
| 36.9381 | 30.12 | 2.43 | 0.85 |
| 39.1682 | 34.03 | 2.30 | 0.96 |

### Example 8 Preparation and characterization of crystalline form A of mucate

500.8 mg of free crystalline form A sample was weighted into a 20 mL glass bottle, 15 mL of acetone/n-heptane (1:9, v:v) was added to dissolve. 129.8 mg of mucic acid (approximately 0.5 equivalent) was added in batches under magnetic stirring (~750 rpm), and was placed at 50 °C for suspending and stirring. Upon reacting overnight, the upper solution of the system was clear and the solid at the bottom was viscous and colloidal. The system was switched to a temperature cycle (50°C to 5°C). After two cycles, the reaction system turns into a white suspension. Upon reacting overnight, the solid was separated by suction filtration and washed with n-heptane. After vacuum drying at room temperature for 2 days, the solid was collected for characterization testing and research.

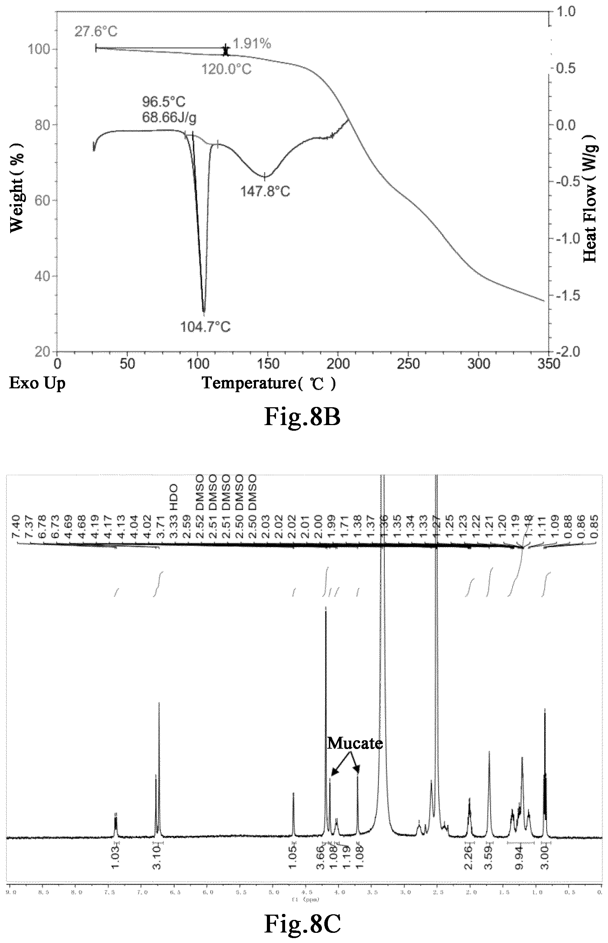

The XRPD results of samples are shown in Fig. 8A. The TGA/DSC profile is shown in Fig. 8B. The ¹H NMR spectrum was obtained from DMSO-d₆ test, and is listed in Fig. 8C. No obvious MTBE residues in the sample was detected.

**Table 12 XRPD peak list of crystalline form A of eliglustat mucate**

| **Position[°2θ]** | **Height [CTS]** | **d-value [Å]** | **Relative Intensity [%]** |
|---|---|---|---|
| 5.3436 | 365.47 | 16.54 | 21.64 |
| 6.4119 | 1689.11 | 13.79 | 100.00 |
| 8.4040 | 482.67 | 10.52 | 28.58 |
| 12.3744 | 158.53 | 7.15 | 9.39 |
| 12.8132 | 78.57 | 6.91 | 4.65 |
| 13.9839 | 205.03 | 6.33 | 12.14 |
| 17.0029 | 140.52 | 5.21 | 8.32 |
| 17.8947 | 78.87 | 4.96 | 4.67 |
| 19.5821 | 117.47 | 4.53 | 6.95 |
| 20.7323 | 432.99 | 4.28 | 25.63 |
| 22.3256 | 68.96 | 3.98 | 4.08 |
| 25.2775 | 74.25 | 3.52 | 4.40 |
| 26.2821 | 65.70 | 3.39 | 3.89 |

### Example 9 Preparation and characterization of crystalline form A/B of malate

Free crystalline form A and 0.5 equivalent of L-malic acid were suspended and stirred in EtOAc for 1 day at room temperature to obtain the sample. The solid was separated by centrifugation and dried under vacuum to obtain crystalline form A of malate. Since the samples deliquesced significantly under room humidity conditions, no other characterization data were collected.

Free crystalline form A and 0.5 equivalent of L-malic acid were suspended and stirred in IPAc/n-heptane (1:5, v:v) for 1 day at room temperature and 2 days at 40 °C, and then the solid was separated by centrifugation. Upon vacuum drying, crystalline form B of malate was obtained. Since the sample absorbed moisture violently under room humidity conditions, subsequent characterization was not conducted.

### Example 10 Preparation and characterization of crystalline form A of glutarate

Crystalline form A of glutarate was obtained by suspending and stirring free crystalline form A and 0.5 equivalents of glutaric acid in IPAc/n-heptane (1:5, v:v) at room temperature for 1 day and at 40 °C for 2 days. The solid was separated by centrifugation and dried under vacuum for subsequent characterization.

The XRPD results of samples are shown in Fig. 7A. The TGA/DSC profile is shown in Fig. 7B. The results show that the weight loss when heated to 150°C is 3.7%, and endothermic signals are observed at 86.7, 101.4 and 177.4 °C (peak temperature). Based on this result, it is speculated that it is a mixture of crystalline form A of glutarate and free crystalline form A. The ¹H NMR spectrum was obtained from DMSO-d₆ test, and is listed in Fig. 7C. The results show that the mole ratio of ligand acid to API is approximately 0.7. No obvious solvent residues was detected.

**Table 13 XRPD peak list of crystalline form A of eliglustat glutarate**

| **Position[°2θ]** | **Height [CTS]** | **d-value [Å]** | **Relative Intensity [%]** |
|---|---|---|---|
| 5.0662 | 1418.03 | 17.44 | 100.00 |
| 6.4008 | 307.38 | 13.81 | 21.68 |
| 7.7509 | 52.33 | 11.41 | 3.69 |
| 10.6264 | 295.26 | 8.33 | 20.82 |
| 10.9169 | 126.50 | 8.10 | 8.92 |
| 11.7381 | 68.37 | 7.54 | 4.82 |
| 13.0613 | 212.37 | 6.78 | 14.98 |
| 14.9440 | 75.98 | 5.93 | 5.36 |
| 15.5301 | 386.23 | 5.71 | 27.24 |
| 16.5758 | 152.93 | 5.35 | 10.78 |
| 17.6208 | 107.60 | 5.03 | 7.59 |
| 17.9076 | 113.64 | 4.95 | 8.01 |
| 18.5881 | 238.35 | 4.77 | 16.81 |
| 18.8915 | 97.60 | 4.70 | 6.88 |
| 19.2551 | 443.09 | 4.61 | 31.25 |
| 19.8783 | 94.58 | 4.47 | 6.67 |
| 20.3740 | 95.99 | 4.36 | 6.77 |
| 21.3087 | 389.28 | 4.17 | 27.45 |
| 21.5201 | 143.67 | 4.13 | 10.13 |
| 21.8679 | 219.19 | 4.06 | 15.46 |
| 22.2079 | 91.03 | 4.00 | 6.42 |
| 22.6535 | 102.03 | 3.93 | 7.20 |
| 23.4297 | 130.58 | 3.80 | 9.21 |
| 23.6661 | 153.90 | 3.76 | 10.85 |
| 24.1975 | 43.46 | 3.68 | 3.06 |
| 26.1820 | 105.42 | 3.40 | 7.43 |
| 27.1414 | 33.64 | 3.29 | 2.37 |
| 27.7509 | 32.18 | 3.21 | 2.27 |

### Example 11 Preparation and characterization of crystalline form A of succinate

Free crystalline form A and 0.5 equivalent of succinic acid were suspended and stirred in IPAc/n-heptane (1:5, v:v) for 1 day at room temperature and 2 days at 40 °C, and the obtained solid was free crystalline form A. After adding an additional 0.25 equivalents of succinic acid, the solution was continued to be suspended and stirred at 40°C for one week. The solid was obtained by centrifugation and dried under vacuum for subsequent characterization.

The XRPD results of samples are shown in Table 14. The TGA/DSC profile show that the weight loss when heated to 120°C is 7.3%, and endothermic signals are observed at 83.7, and 169.9°C (peak temperature). The ¹H NMR spectrum was obtained from DMSO-d₆ test. The results show that the mole ratio of ligand acid to API is approximately 0.6. No obvious solvent residues was detected.

**Table 14 XRPD peak list of crystalline form A of eliglustat succinate**

| **Position[°2θ]** | **Height [CTS]** | **d-value [Å]** | **Relative Intensity [%]** |
|---|---|---|---|
| 7.6107 | 344.45 | 11.62 | 100.00 |
| 10.2809 | 97.98 | 8.60 | 28.45 |
| 11.4613 | 29.52 | 7.72 | 8.57 |
| 12.3601 | 28.56 | 7.16 | 8.29 |
| 13.3527 | 67.18 | 6.63 | 19.50 |
| 14.7486 | 276.90 | 6.01 | 80.39 |
| 16.1281 | 67.68 | 5.50 | 19.65 |
| 18.1308 | 155.85 | 4.89 | 45.25 |
| 19.2851 | 149.96 | 4.60 | 43.54 |
| 20.9202 | 52.34 | 4.25 | 15.20 |
| 22.1132 | 119.54 | 4.02 | 34.71 |
| 23.2640 | 108.01 | 3.82 | 31.36 |
| 24.3480 | 186.79 | 3.66 | 54.23 |

### Example 12 Preparation and characterization of crystalline form A of phosphate

Free crystalline form A and 1 equivalent of phosphoric acid (85%) were suspended and stirred in MTBE at room temperature for 1 day to obtain the sample. The solid was separated by centrifugation and dried under vacuum for subsequent characterization.

The XRPD results of samples are shown in Table 15. The TGA/DSC profile show that the weight loss when heated to 100°C is 4.4%, and endothermic signals are observed at 79.7, 106.4, and 131.5°C (peak temperature). The ¹H NMR spectrum was obtained from DMSO-d₆ test. IC/HPLC test results show that the mole ratio of ligand acid to API is approximately 1.4.

**Table 15 XRPD peak list of crystalline form A of eliglustat phosphate**

| **Position[°2θ]** | **Height [CTS]** | **d-value [Å]** | **Relative Intensity [%]** |
|---|---|---|---|
| 3.2097 | 2493.28 | 27.53 | 99.08 |
| 6.2873 | 2516.52 | 14.06 | 100.00 |
| 7.6982 | 127.28 | 11.48 | 5.06 |
| 9.4151 | 87.33 | 9.39 | 3.47 |
| 11.9267 | 39.96 | 7.42 | 1.59 |
| 12.5600 | 1533.80 | 7.05 | 60.95 |
| 14.5840 | 241.49 | 6.07 | 9.60 |
| 15.2397 | 66.64 | 5.81 | 2.65 |
| 15.7125 | 251.57 | 5.64 | 10.00 |
| 18.8582 | 118.54 | 4.71 | 4.71 |
| 20.4244 | 102.10 | 4.35 | 4.06 |
| 21.5024 | 115.89 | 4.13 | 4.61 |
| 22.0128 | 268.19 | 4.04 | 10.66 |
| 22.9422 | 79.03 | 3.88 | 3.14 |
| 24.8207 | 109.00 | 3.59 | 4.33 |
| 25.9880 | 42.85 | 3.43 | 1.70 |
| 28.4828 | 57.54 | 3.13 | 2.29 |
| 34.9449 | 120.34 | 2.57 | 4.78 |
| 38.2229 | 70.66 | 2.35 | 2.81 |

### Example 13 Preparation and characterization of crystalline form A of hydrochloride

Free crystalline form A sample was suspended and stirred with an equivalent amount of hydrochloric acid in EtOAc at room temperature to obtain a clear solution, which was then added with n-heptane antisolvent to form a colloidal substance. The colloidal substance sample was transferred to a temperature cycle of 50 °C to 5 °C. After 2 days, a solid sample was obtained, which was centrifuged and vacuum dried for subsequent characterization.

The XRPD results of samples are shown in Table 16. The TGA/DSC profile show that the weight loss when heated to 100°C is 11.7%, and endothermic signals are observed at 48.6, and 90.3°C (peak temperature). Due to insufficient sample size from screening, NMR testing was not conducted. IC/HPLC test results show that the mole ratio of ligand acid to API is approximately 1.0.

**Table 16 XRPD peak list of crystalline form A of eliglustat phosphate**

| **Position[°2θ]** | **Height [CTS]** | **d-value [Å]** | **Relative Intensity [%]** |
|---|---|---|---|
| 6.8700 | 540.99 | 12.87 | 100.00 |
| 12.1637 | 128.30 | 7.28 | 23.71 |
| 12.9771 | 58.05 | 6.82 | 10.73 |
| 13.8519 | 189.90 | 6.39 | 35.10 |
| 15.1571 | 41.73 | 5.85 | 7.71 |
| 17.2576 | 36.12 | 5.14 | 6.68 |
| 18.6147 | 61.80 | 4.77 | 11.42 |
| 19.3344 | 88.13 | 4.59 | 16.29 |
| 20.6404 | 256.28 | 4.30 | 47.37 |
| 21.1449 | 78.16 | 4.20 | 14.45 |
| 22.2020 | 32.62 | 4.00 | 6.03 |
| 24.1327 | 212.09 | 3.69 | 39.20 |
| 24.6323 | 230.72 | 3.61 | 42.65 |
| 27.0694 | 51.00 | 3.29 | 9.43 |
| 27.6616 | 45.51 | 3.22 | 8.41 |
| 31.1475 | 25.77 | 2.87 | 4.76 |
| 37.0269 | 28.35 | 2.43 | 5.24 |

### Example 14 Preparation and characterization of crystalline form A of fumarate

Free crystalline form A sample and 0.5 equivalents of fumaric acid were suspended and stirred at room temperature in EtOH/n-heptane (1:1, v:v) to obtain a clear solution, after adding n-heptane antisolvent, an oily substance was obtained. Upon exposure evaporation at room temperature, a solid was obtained, which was dried under vacuum and used for subsequent characterization.

The XRPD results of samples are shown in Table 17. The TGA/DSC profile show that the weight loss when heated to 100°C is 9.1%, and endothermic signals are observed at 54.6, 86.8, and 134.1°C (peak temperature). The ¹H NMR spectrum was obtained from DMSO-d₆ test. The results show that the mole ratio of ligand acid to API is approximately 0.5. No obvious solvent residues was detected.

**Table 17 XRPD peak list of crystalline form A of eliglustat fumarate**

| **Position[°2θ]** | **Height [CTS]** | **d-value [Å]** | **Relative Intensity [%]** |
|---|---|---|---|
| 3.0912 | 694.86 | 28.58 | 100.00 |
| 3.3891 | 656.28 | 26.07 | 94.45 |
| 3.9026 | 543.61 | 22.64 | 78.23 |
| 5.0364 | 351.61 | 17.55 | 50.60 |
| 6.0735 | 220.62 | 14.55 | 31.75 |
| 6.5703 | 134.66 | 13.45 | 19.38 |
| 7.7255 | 428.06 | 11.44 | 61.60 |
| 10.4677 | 43.14 | 8.45 | 6.21 |
| 13.4851 | 74.41 | 6.57 | 10.71 |
| 14.8145 | 272.36 | 5.98 | 39.20 |
| 16.9889 | 24.11 | 5.22 | 3.47 |
| 17.8749 | 40.20 | 4.96 | 5.79 |
| 19.6942 | 90.97 | 4.51 | 13.09 |
| 21.0953 | 171.04 | 4.21 | 24.61 |
| 22.3015 | 114.80 | 3.99 | 16.52 |
| 23.5948 | 69.72 | 3.77 | 10.03 |
| 24.5585 | 104.97 | 3.62 | 15.11 |
| 30.5276 | 22.99 | 2.93 | 3.31 |

### Example 15 Dynamic solubility test

The dynamic solubility of crystalline form B/crystalline form C of 1,5-naphthalene disulfonate, crystalline form A of oxalate, crystalline form A of tartrate and free crystalline form A in water and three biological solvents were evaluated.

In the test, the solid dosage concentration of ~10 mg/mL (~40 mg solid into 4 mL of solvent) was rotated and mixed at 37°C, and the solubility of each sample in four systems: water, SGF, FaSSIF and FeSSIF1 was measured at different time points (1, 4 and 24 hours). After sampling at each time point, the samples were centrifugally filtered (0.45 µm PTFE filter head), the free salt concentration and pH value in the filtrate were measured, and the solid samples after centrifugation were tested for XRPD. The solubility test results are summarized in Table 18.

**Table 18 Summary of solubility test results**

| | | **1 hr** | | | **4 hrs** | | | **12 hrs** | | |
|---|---|---|---|---|---|---|---|---|---|---|
| **Sample** | **Solvent** | **S** | **pH** | **FC** | **S** | **pH** | **FC** | **S** | **pH** | **FC** |
| Crystalline form B of 1,5-naphthalene disulfonate | H₂O | 1.9 | 7.67 | Yes | 2.0 | 7.67 | Yes | 2.0 | 7.75 | Yes |
| | SGF | 2.7 | 1.84 | Yes | 2.5 | 1.75 | Yes | 2.3 | 1.77 | Yes |
| | FaSSIF | 2.7 | 6.52 | Yes | 2.7 | 6.51 | Yes | 2.6 | 6.52 | Yes |
| Crystalline form C of 1,5-naphthalene disulfonate | H₂O | 2.0 | 7.39 | No | 1.9 | 7.33 | No | 2.0 | 7.45 | No |
| | SGF | 2.6 | 1.80 | No | 2.4 | 1.88 | No | 2.6 | 1.89 | No |
| | FaSSIF | 2.9 | 6.48 | No | 2.8 | 6.48 | No | 3.0 | 6.49 | No |
| Crystalline form A of oxalate | H₂O | 6.8 | 7.07 | No | 7.8 | 7.51 | No | 8.0 | 7.30 | No |
| | SGF | ≥8.4 | 2.19 | NA | ≥8.5 | 2.17 | NA | ≥8.2 | 2.05 | NA |
| | FaSSIF | ≥8.1 | 6.52 | NA | ≥8.2 | 6.48 | NA | ≥8.1 | 6.47 | NA |
| Crystalline form A of tartrate | H₂O | ≥8.0 | 4.99 | NA | ≥8.1 | 5.13 | NA | ≥9.5 | 5.09 | NA |
| | SGF | ≥8.0 | 2.80 | NA | ≥8.0 | 2.82 | NA | ≥8.1 | 2.89 | NA |
| | FaSSIF | ≥7.9 | 6.39 | NA | ≥7.7 | 6.38 | NA | ≥7.6 | 6.38 | NA |
| Free crystalline form A | H₂O | 0.12 | 9.24 | No | 0.13 | 9.03 | No | 0.17 | 8.80 | No |
| | SGF | 6.6 | 7.27 | No | 6.7 | 7.26 | No | 6.5 | 7.30 | No |
| | FaSSIF | 4.4 | 7.31 | No | 4.4 | 7.29 | No | 4.2 | 7.30 | No |

| | | | | | | | | | | |
|---|---|---|---|---|---|---|---|---|---|---|
| S: Solubility (mg/mL, free concentration); FC: Crystalline form transformation; NA: Since the sample was clear, no XRPD characterization was conducted; | | | | | | | | | | |

### Example 16 Evaluation of hygroscopicity

The hygroscopicity of crystalline form B/crystalline form C of 1,5-naphthalene disulfonate, crystalline form A of oxalate, crystalline form A of tartrate and free crystalline form A were evaluated by DVS.

The results show that when the humidity is higher than 80% RH at 25°C, crystalline form B of 1,5-naphthalene disulfonate would undergo significant hygroscopic weight gain and changed to crystalline form C after the DVS test; the hygroscopic weight gain of crystalline form C of 1,5-naphthalene disulfonate at 25°C/80%RH is 0.33 wt%, and there was no change in crystalline form after DVS test; the hygroscopic weight gain of crystalline form A of oxalate at 25°C/80%RH is 7.64 wt%. Significant weight loss was observed when the humidity is lower than 10%RH, and there was no change in crystalline form after DVS test. The crystalline form A sample of tartrate is slightly hygroscopic, with a hygroscopic weight gain of 1.11 wt% at 25°C/80% RH. Free crystalline form A sample has almost no hygroscopicity, and both samples did not change in crystalline form after DVS test. DVS test results are listed in Table 19.

**Table 19 Results of hygroscopicity evaluation**

| **Crystalline form** | **Hygroscopic weight gain at 25°C/80%RH** | **Transformation of crystalline form** |
|---|---|---|
| Crystalline form B of 1,5-naphthalene disulfonate | 0.59% | Change to crystalline form C |
| Crystalline form C of 1,5-naphthalene disulfonate | 0.33% | No change |
| Crystalline form A of oxalate | 7.64% | No change |
| Crystalline form A of tartrate | 1.11% | No change |
| Free crystalline form A | 0.01% | No change |

### Example 17 Solid state stability assessment

After leaving the crystalline form C of 1,5-naphthalene disulfonate in the exposure evaporation at 25°C/60%RH and 40°C/75%RH for 1 week, 3 weeks and 4 weeks. The physical and chemical stability of the sample was tested by XRPD and HPLC, and the stability data of crystalline form A of tartrate and free crystalline form A at 1 week, 2 weeks and 4 weeks were used as a reference. At the same time, the stability of crystalline form B of 1,5-naphthalene disulfonate and crystalline form A of oxalate was tested under the conditions of 25°C/60%RH and 40°C/75%RH for 1 week. The results showed that no change in crystalline form or decrease in HPLC purity was observed in the samples of crystalline form C of 1,5-naphthalene disulfonate, crystalline form A of tartrate, and free crystalline form A after being left exposed for 4 weeks under corresponding conditions. With regard to the samples of crystalline form B of 1,5-naphthalene disulfonate and crystalline form A of oxalate, no decrease in HPLC purity was observed after 1 week of exposure evaporation under corresponding conditions. However, the crystalline form B of 1,5-naphthalene disulfonate partially transformed into the crystalline form C after being left exposed for a week at 40°C/75%RH.

### Example 18 Study of drug efficacy

Plasma levels of glucosylceramide (GL-1) are considered a biomarker for substrate reduction therapy (SRT) in Gaucher's patients and a surrogate biomarker for SRT in Fabry's patients to assess the biological effects of the newly discovered salts in preclinical species.

While various embodiments have been described, the scope of the disclosure will be defined by the appended claims rather than by the specific embodiments that have been shown by way of example. The contents of all references cited throughout this application (including literature references, published patents, published patent applications and co-pending patent applications) are hereby expressly incorporated by reference in their entirety. Unless otherwise specified, all technical and scientific terms used in this application have the meaning commonly understood by those skilled in the art.

## Claims

1. A pharmaceutically acceptable salt of eliglustat, **characterized in that** the solubility of the pharmaceutically acceptable salt in water and simulated gastric fluid is less than or equal to 6.0 mg/mL.

2. A pharmaceutically acceptable salt of eliglustat, **characterized in that** the pharmaceutically acceptable salts are naphthalene disulfonate, mucate, glutarate.

3. A pharmaceutically acceptable salt of eliglustat, **characterized in that** the naphthalene disulfonate is 1,5-naphthalene disulfonate.

4. The pharmaceutically acceptable salt of eliglustat according to claim 3, wherein the molar ratio of 1,5-naphthalene disulfonic acid and eliglustat is 1:1 or 1:2.

5. The pharmaceutically acceptable salt of eliglustat according to claim 4, wherein the pharmaceutically acceptable salts are the hydrate or unsolvate of 1,5-naphthalene disulfonate.

6. The pharmaceutically acceptable salt of eliglustat according to claim 5, wherein the hydrate of 1,5-naphthalene disulfonate is hemihydrate, monohydrate, and dihydrate.

7. The pharmaceutically acceptable salt of eliglustat according to any one of claims 1-6, wherein the salt is crystalline form D of eliglustat 1,5-naphthalene disulfonate with X-ray powder diffraction peaks at 2θ angles (± 0.2°) of 4.9°, 5.9°, and 18.7°.

8. The pharmaceutically acceptable salt of eliglustat according to claim 7, wherein the crystalline form D of the eliglustat 1,5-naphthalene disulfonate further comprises X-ray powder diffraction peaks at 2θ angles (± 0.2°) of 7.0°, 10.4°, and 24.7°.

9. The pharmaceutically acceptable salt of eliglustat according to claim 8, wherein the crystalline form D of the eliglustat 1,5-naphthalene disulfonate further comprises X-ray powder diffraction peaks at 2θ angles (± 0.2°) of 14.2° and 16.2°.

10. The pharmaceutically acceptable salt of eliglustat according to any one of claims 1-6, wherein the salt is crystalline form D of the eliglustat 1,5-naphthalene disulfonate with X-ray powder diffraction pattern that is substantially similar to Fig. 3A.

11. The pharmaceutically acceptable salt of eliglustat according to any one of claims 1-6, wherein the salt is crystalline form B of eliglustat 1,5-naphthalene disulfonate with X-ray powder diffraction peaks at 2θ angles (± 0.2°) of 7.3°, 14.6°, and 6.5°.

12. The pharmaceutically acceptable salt of eliglustat according to claim 11, wherein the crystalline form B of the eliglustat 1,5-naphthalene disulfonate further comprises X-ray powder diffraction peaks at 2θ angles (± 0.2°) of 22.8°, 21.0°, and 20.8°.

13. The pharmaceutically acceptable salt of eliglustat according to claim 12, wherein the crystalline form B of the eliglustat 1,5-naphthalene disulfonate further comprises X-ray powder diffraction peaks at 2θ angles (± 0.2°) of 13.1°, 3.3° and 15.1°.

14. The pharmaceutically acceptable salt of eliglustat according to claim 13, wherein the crystalline form B of the eliglustat 1,5-naphthalene disulfonate has an X-ray powder diffraction pattern that is substantially similar to Fig. 4A.

15. The pharmaceutically acceptable salt of eliglustat according to any one of claims 1-6, wherein the salt is crystalline form C of eliglustat 1,5-naphthalene disulfonate with X-ray powder diffraction peaks at 2θ angles (± 0.2°) of 9.4°, 13.6°, 20.1°, and 12.1°.

16. The pharmaceutically acceptable salt of eliglustat according to claim 15, wherein the crystalline form C of the eliglustat 1,5-naphthalene disulfonate further comprises X-ray powder diffraction peaks at 2θ angles (± 0.2°) of 24.3°, 12.8°, and 19.6°.

17. The pharmaceutically acceptable salt of eliglustat according to claim 16, wherein the crystalline form C of the eliglustat 1,5-naphthalene disulfonate further comprises X-ray powder diffraction peaks at 2θ angles (± 0.2°) of 6.2°, and 14.0°.

18. The pharmaceutically acceptable salt of eliglustat according to claim 15, wherein the crystalline form C of the eliglustat 1,5-naphthalene disulfonate has an X-ray powder diffraction pattern that is substantially similar to Fig. 5A.

19. A pharmaceutically acceptable salt of eliglustat, wherein the salt is crystalline form A of eliglustat oxalate with X-ray powder diffraction peaks at 2θ angles (± 0.2°) of 7.5°, 15.5°, and 19.0°.

20. The pharmaceutically acceptable salt of eliglustat according to claim 19, wherein the crystalline form A of the eliglustat oxalate further comprises X-ray powder diffraction peaks at 2θ angles (± 0.2°) of 10.0°, 22.3°, and 23.3°.

21. The pharmaceutically acceptable salt of eliglustat according to claim 20, wherein the crystalline form A of the eliglustat oxalate further comprises X-ray powder diffraction peaks at 2θ angles (± 0.2°) of 12.8°, 18.3°, and 20.7°.

22. The pharmaceutically acceptable salt of eliglustat according to claim 19, wherein the crystalline form A of the eliglustat oxalate has an X-ray powder diffraction pattern that is substantially similar to Fig. 6A.

23. The pharmaceutically acceptable salt of eliglustat according to any one of claims 1-2, wherein the salt is crystalline form A of eliglustat glutarate with X-ray powder diffraction peaks at 2θ angles (± 0.2°) of 5.1°, 19.3°, and 21.3°.

24. The pharmaceutically acceptable salt of eliglustat according to claim 23, wherein the crystalline form A of the eliglustat glutarate further comprises X-ray powder diffraction peaks at 2θ angles (± 0.2°) of 15.5°, 6.4°, and 10.6°.

25. The pharmaceutically acceptable salt of eliglustat according to claim 24, wherein the crystalline form A of the eliglustat glutarate further comprises X-ray powder diffraction peaks at 2θ angles (± 0.2°) of 18.6°, 21.9°, and 13.1°.

26. The pharmaceutically acceptable salt of eliglustat according to claim 23, wherein the crystalline form A of the eliglustat glutarate has an X-ray powder diffraction pattern that is substantially similar to Fig. 7A.

27. The pharmaceutically acceptable salt of eliglustat according to any one of claims 1-2, wherein the salt is crystalline form A of eliglustat mucate with X-ray powder diffraction peaks at 2θ angles (± 0.2°) of 6.4°, 8.4°, and 20.7°.

28. The pharmaceutically acceptable salt of eliglustat according to claim 27, wherein the crystalline form A of the eliglustat mucate further comprises X-ray powder diffraction peaks at 2θ angles (± 0.2°) of 5.3°, 14.0°, and 12.4°.

29. The pharmaceutically acceptable salt of eliglustat according to claim 28, wherein the crystalline form A of the eliglustat mucate further comprises X-ray powder diffraction peaks at 2θ angles (± 0.2°) of 17.0°, 19.6°, and 17.9°± 0.2°.

30. The pharmaceutically acceptable salt of eliglustat according to claim 27, wherein the crystalline form A of the eliglustat mucate has an X-ray powder diffraction pattern that is substantially similar to Fig. 8A.

31. The pharmaceutically acceptable salt of eliglustat according to any one of claims 7-30, wherein the compound is at least 60% by weight of the monomorph form, at least 70% by weight of the monomorph form, at least 80% by weight of the monomorph form, at least 90% by weight of the monomorph form, at least 95% by weight of the monomorph form, or at least 99% by weight of the monomorph form.

32. A pharmaceutical composition comprising a pharmaceutically acceptable salt of eliglustat according to any one of claims 1-4, or hydrate of eliglustat 1,5-naphthalene disulfonate according to any one of claims 5-6, or crystalline form D of eliglustat 1,5-naphthalene disulfonate according to any one of claims 7-10, or crystalline form B of eliglustat 1,5-naphthalene disulfonate according to any one of claims 11-14, or crystalline form C of eliglustat 1,5-naphthalene disulfonate according to any one of claims 15-18, or crystalline form A of eliglustat oxalate according to any one of claims 19-22, or crystalline form A of eliglustat glutarate according to any one of claims 23-26, or crystalline form A of eliglustat mucate according to any one of claims 31-34, and the pharmaceutically acceptable carrier.

33. A method for preparing crystalline form D of eliglustat 1,5-naphthalene disulfonate, **characterized in that** eliglustat free base and 1-1.1 equivalents of 1,5-naphthalene disulfonic acid are dissolved in methyl tert-butyl ether, the mixture system is magnetically stirred at room temperature for 1-3 days and then centrifuged, the obtained solid is dried under vacuum at room temperature overnight.

34. A method for preparing crystalline form B of eliglustat 1,5-naphthalene disulfonate, **characterized in that** the eliglustat free base and 0.5 equivalents of 1,5-naphthalene disulfonic acid are dissolved in tetrahydrofuran/n-heptane (1:9, v:v) for continuous suspending and stirring at a temperature of 20-40°C, and the precipitated solid is dried.

35. A method for preparing crystalline form C of eliglustat 1,5-naphthalene disulfonate, **characterized in that** the crystalline form B of eliglustat 1,5-naphthalene disulfonate is added to H₂O, and stirred at room temperature, the solid is separated and dried with calcium oxide.

36. A method for preparing crystalline form A of eliglustat oxalate, **characterized in that** the eliglustat free base and 0.5 equivalents of oxalic acid are dissolved in methyl tert-butyl ether for continuously suspending and stirring at 15-50°C, and the precipitated solid is dried.

37. A method for preparing crystalline form A of eliglustat glutarate, **characterized in that** the eliglustat free base and 0.5 equivalents of glutaric acid are dissolved in isopropyl acetate/n-heptane (1:5, v:v) for continuously suspending and stirring at 20-40°C, and the precipitated solid is dried.

38. A method for preparing crystalline form A of eliglustat murate, **characterized in that** the eliglustat free base and 0.5 equivalents of mucic acid are dissolved in acetone/n-heptane (1:9, v:v) for continuously suspending and stirring at 35-45°C, and the precipitated solid is dried to obtain the crystalline form A of eliglustat murate.

39. Use of pharmaceutically acceptable salt of eliglustat according to any one of claims 1-4, or hydrate of eliglustat 1,5-naphthalene disulfonate according to any one of claims 5-6, or crystalline form D of eliglustat 1,5-naphthalene disulfonate according to any one of claims 7-10, or crystalline form B of eliglustat 1,5-naphthalene disulfonate according to any one of claims 11-14, or crystalline form C of eliglustat 1,5-naphthalene disulfonate according to any one of claims 15-18, or crystalline form A of eliglustat oxalate according to any one of claims 19-22, or crystalline form A of eliglustat glutarate according to any one of claims 23-26, or crystalline form A of eliglustat mucate according to any one of claims 27-30 in the treatment of Gaucher's disease, Fabry's disease, and polycystic kidney disease.

40. The use of claim 39, wherein the patient with the disease is an extensive, intermediate or poor metabolizer of CYP2D6.

41. The used of claim 39 or 40, wherein Gaucher's disease is Type I Gaucher's disease and the polycystic kidney disease is autosomal dominant polycystic kidney disease.

42. Use of pharmaceutically acceptable salt of eliglustat according to any one of claims 1-4, or hydrate of eliglustat 1,5-naphthalene disulfonate according to any one of claims 5-6, or crystalline form D of eliglustat 1,5-naphthalene disulfonate according to any one of claims 7-10, or crystalline form B of eliglustat 1,5-naphthalene disulfonate according to any one of claims 11-14, or crystalline form C of eliglustat 1,5-naphthalene disulfonate according to any one of claims 15-18, or crystalline form A of eliglustat oxalate according to any one of claims 19-22, or crystalline form A of eliglustat glutarate according to any one of claims 23-26, or crystalline form A of eliglustat mucate according to any one of claims 27-30 in the manufacture of a medicament in treating of Gaucher's disease, Fabry's disease, and polycystic kidney disease.

43. The use of claim 42, wherein the patient with the disease is an extensive, intermediate or poor metabolizer of CYP2D6.

44. The used of claim 42 or 43, wherein Gaucher's disease is Type I Gaucher's disease and the polycystic kidney disease is autosomal dominant polycystic kidney disease.
